(19) Europäisches Patentamt
European Patent Office
Office européen des brevets

(11) Veröffentlichungsnummer: **0 136 974**
**A2**

(12) # EUROPÄISCHE PATENTANMELDUNG

(21) Anmeldenummer: **84810383.4**

(22) Anmeldetag: **06.08.84**

(51) Int. Cl.⁴: **C 07 D 237/24**
A 01 N 43/58

(30) Priorität: **10.08.83 CH 4364/83**

(43) Veröffentlichungstag der Anmeldung:
**10.04.85 Patentblatt 85/15**

(84) Benannte Vertragsstaaten:
**AT BE CH DE FR IT LI NL SE**

(71) Anmelder: **CIBA-GEIGY AG**
**Postfach**
**CH-4002 Basel(CH)**

(72) Erfinder: **Dürr, Dieter, Dr.**
**Brändelistalweg 16**
**CH-4103 Bottmingen(CH)**

(72) Erfinder: **Tobler, Hans, Dr.**
**Baselmattweg 157**
**CH-4123 Allschwil(CH)**

(54) **Pyridazinylcarbonsäure-derivate mit gametozider Wirkung.**

(57) Neue Pyridazinylcarbonsäure-derivate der Formel

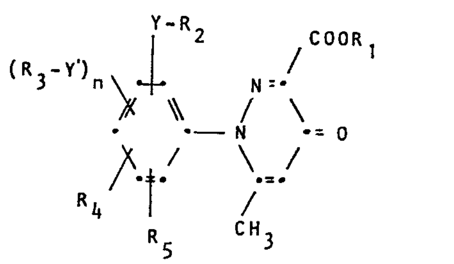

(I)

worin
$R_1$ Wasserstoff, ein Kation oder $C_1$-$C_8$-Alkyl,
Y und Y' unabhängig voneinander ein Sauerstoff- oder Schwefelatom oder eine Sulfinyl- oder Sulfonylgruppe,
$R_2$ und $R_3$ unabhängig voneinander eine ein- oder mehrfach durch Halogen substituierte $C_1$-$C_{12}$-Alkyl- oder $C_1$-$C_{12}$-Alkenylgruppe,
$R_4$ und $R_5$ unabhängig voneinander Wasserstoff, Halogen, Nitro, Cyan,
$C_1$-$C_6$-Alkoxy oder unsubstituiertes oder ein- oder mehrfach durch Halogen substituiertes $C_1$-$C_6$-Alkyl und n 0 oder 1 bedeutet.

Die Verbindungen weisen gametozide Wirkung auf und können zur Beeinflussung des generativen Pflanzenwachstums und zur Erzeugung männlich steriler Pflanzen verwendet werden.

EP 0 136 974 A2

0136974



CIBA-GEIGY AG
Basel (Schweiz)

5-14535/+

# Pyridazinylcarbonsäure-derivate mit gametozider Wirkung

Die vorliegende Erfindung betrifft neue Pyridazinylcarbonsäure-derivate mit gametozider Wirkung. Sie betrifft ebenfalls die Herstellung der neuen Verbindungen und Mittel, welche diese Pyridazinylcarbonsäure-derivate als Wirkstoff enthalten. Sie betrifft auch die Verwendung dieser Mittel und der zugrundeliegenden Aktivsubstanzen zur Stimulierung des generativen Pflanzenwachstums, insbesondere deren Verwendung zur Erzeugung männlich steriler Pflanzen (Einsatz als Gametozide). Sie betrifft ferner die Verwendung der Pyridazinylcarbonsäure-derivate zur Steuerung der Blütenbildung und der damit verbundenen Sekundäreffekte.

Die Pyridazinylcarbonsäure-derivate entsprechen der Formel I

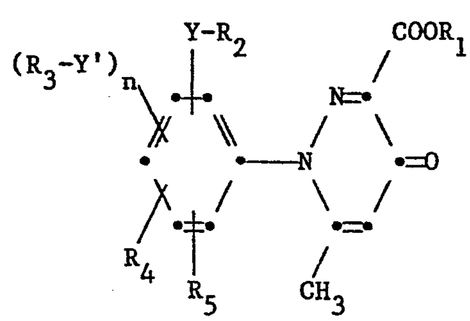

(I)

worin

$R_1$ Wasserstoff, ein Kation oder $C_1$-$C_6$-Alkyl,

Y und Y' unabhängig voneinander ein Sauerstoff- oder Schwefelatom oder eine Sulfinyl- oder Sulfonylgruppe,

$R_2$ und $R_3$ unabhängig voneinander eine ein- oder mehrfach durch Halogen substituierte $C_1$-$C_{12}$-Alkyl- oder $C_1$-$C_{12}$-Alkenylgruppe, $R_4$ und $R_5$ unabhängig voneinander Wasserstoff, Halogen, Nitro, Cyan, $C_1$-$C_6$-Alkoxy oder unsubstituiertes oder ein- oder mehrfach durch Halogen substituiertes $C_1$-$C_6$-Alkyl und n 0 oder 1 bedeutet.

Unter einem Kation ist beispielsweise das anorganische Kation eines Elements der ersten bis vierten Hauptgruppe des Periodensystems zu verstehen. Typische Vertreter sind die Alkalimetalle wie Lithium, Natrium, Kalium oder die Erdalkalimetalle wie Magnesium, Calcium, Barium oder Elemente wie Aluminium, Zinn oder Blei. Darunter ist ferner das Kation eines Elements der ersten bis achten Nebengruppe zu verstehen, wie z.B. von Chrom, Mangan, Eisen, Kobalt, Nickel, Kupfer, Zink, Silber oder Quecksilber. Bevorzugt sind Alkali- und Erdalkalikationen sowie die Kationen der Elemente aus der dritten und vierten Periode des Periodensystems.

Als Ammoniumionen kommen z.B. in Frage: $NH_4$, $NH(Alkyl)_3$, $NH_2(Alkyl)_2$ und $NH_3(Alkyl)$ wie $NH(CH_3)_3$, $NH(C_2H_5)_3$, $NH_2(CH_3)_2$, $NH_2(C_3H_7\text{-}n)_2$, $NH_3CH_3$, $NH_3C_4H_9$-n oder quaternäre Ammoniumionen wir Tetraethyl-, Tetrapropyl-, Tetrabutyl-, Tetrapentyl-, Tetrahexyl-, Tetraheptyl-, Tetraoctyl-, Tetranonyl-, Tetradecyl-, Methyltributyl-, Dimethyldibutyl-, Trimethylbutyl-, Methyltrioctyl-, Benzyltrimethyl-, Benzyltriethyl-, Benzyltripropyl-, Benzyltributyl-, Benzyldimethylhexadecyl-, Benzyldiethylhexadecyl-, Diisobutyl-cresoxyethyldimethylbenzyl-, Trimethylphenyl-, Diphenyldimethyl-, Butyltripropyl-, Tributylphenyl- oder Tricaprylmethylammonium. Als Hydraziniumionen kommen unsubstituierte und substituierte Hydraziniumverbindungen in Frage wie beispielsweise $NH_2NH_3$, $NH_2N(Alkyl)_3$, $NH_2NH(Alkyl)_2$, $NH_2NH_2$-(Alkyl), und als Guanidiniumionen beispielsweise $NH_2\text{-}C(=NH)\text{-}NH_3^{\oplus}$ oder $NH_2\text{-}NH\text{-}C(=NH)\text{-}NH\text{-}NH_3^{\oplus}$.

Unter den Ammoniumkationen sind neben dem $NH_4$ insbesondere solche des Typs $NH_{(4-a)}(Niederalkyl)_a$ mit a = 1, 2, 3, 4 bevorzugt und unter diesen vor allem symmetrische Tetraalkylammoniumionen, wie $N(CH_3)_4$, $N(C_2H_5)_4$, $N(C_4H_9-n)_4$.

Unter Alkyl als Substituent oder Teil eines Substituenten sind je nach Zahl der angegebenen Kohlenstoffatome beispielsweise folgende Gruppen zu verstehen: Methyl, Aethyl, n-Propyl, n-Butyl, n-Pentyl, n-Hexyl, n-Heptyl, n-Octyl, n-Nonyl, n-Decyl, n-Undecyl oder n-Dodecyl sowie ihre Isomeren, wie z.B. Isopropyl, Isobutyl, tert.-Butyl.

Unter Alkenyl als Substituent oder Teil eines Substituenten sind im Rahmen der jeweils angegebenen Anzahl von Kohlenstoffatomen alle geradkettigen oder verzweigten Alkenylgruppen zu verstehen, wie beispielsweise Vinyl, 1-Propenyl, Allyl, Isopropenyl, 2-Butenyl oder 2-Pentyl.

Halogenierte Alkyl- und Alkenylgruppen als Substituenten oder Teile von Substituenten können ein- oder mehrfach durch gleiche oder verschiedene Halogenatome substituiert sein. Bevorzugt sind Alkyl- und Alkenylgruppen mit 1 bis 6, insbesondere 1 bis 4 Kohlenstoffatomen, welche durch mindestens zwei Halogenatome substituiert sind, wobei alle Halogenatome untereinander gleich sind. Bevorzugt sind vor allem fluorierte Alkylgruppen.

Beispiele für halogenierte Alkylgruppen sind $CH_2J$, $CHCl_2$, $CCl_3$, $CH_2Cl$, $CH_2Br$, $CF_2Cl$, $CH_2CH_2CH_2Cl$, $CH_2CF_3$, $CH_2CCl_3$, $CCl_2CHCl_2$, $CH_2CH_2CHCl_2$ und insbesondere $CF_3$, $CHF_2$, $CF_2CF_3$, $CF_2CHFCl$ und $CF_2CHF_2$. Beispiele für halogenierte Alkenylgruppen sind $CH_2CCl=CCl_2$, $CH=CCl_2$ und insbesondere $CCl=CCl_2$ und $CCl=CHCl$.

Unter Halogen als Substituent oder Teil eines Substituenten sind Fluor, Chlor, Brom oder Jod zu verstehen.

Eine bevorzugte Gruppe von Wirkstoffen bilden Verbindungen der Formel I, in denen $R_1$ Wasserstoff, ein Metall-, Ammonium- oder Hydraziniumkation oder $C_1$-$C_4$-Alkyl, Y und Y' unabhängig voneinander ein Sauerstoff- oder Schwefelatom oder eine Sulfinyl- oder Sulfonylgruppe, $R_2$ und $R_3$ unabhängig voneinander eine ein- oder mehrfach durch Halogen substituierte $C_1$-$C_{12}$-Alkyl- oder $C_1$-$C_{12}$-Alkenylgruppe, $R_4$ und $R_5$ unabhängig voneinander Wasserstoff, Halogen, Nitro oder $C_1$-$C_6$-Alkyl und n 0 oder 1 bedeuten, und von diesen Verbindungen insbesondere diejenigen, in denen $R_1$ Wasserstoff, ein Alkalimetall- oder Erdalkalimetallkation, das Ammoniumkation oder $C_1$-$C_4$-Alkyl, Y und Y' unabhängig voneinander ein Sauerstoff- oder Schwefelatom oder eine Sulfonylgruppe, $R_2$ und $R_3$ unabhängig voneinander eine durch mindestens zwei Fluoratome substituierte $C_1$-$C_3$-Alkylgruppe oder eine durch mindestens zwei Chloratome substituierte $C_2$-$C_4$-Alkenylgruppe, $R_4$ Wasserstoff, Halogen, Nitro oder $C_1$-$C_4$-Alkyl, $R_5$ Wasserstoff und n 0 oder 1 bedeuten.

Im Vordergrund des Interesses stehen Verbindungen der Formel I, in denen $R_1$ Wasserstoff, das Natrium-, Kalium-, Magnesium- oder Ammoniumkation oder $C_1$-$C_4$-Alkyl, Y ein Sauerstoff- oder Schwefelatom oder eine Sulfonylgruppe, $R_2$ Trifluormethyl, Difluormethyl, 1,1,2,2,2-Pentafluoräthyl, 1,1,2,2-Tetrafluoräthyl, 1,2-Dichlorvinyl oder 1,2,2-Trichlorvinyl, Y' ein Sauerstoffatom, $R_3$ Difluormethyl, $R_4$ Wasserstoff, Chlor, Nitro oder Methyl, $R_5$ Wasserstoff und n 0 oder 1, bevorzugt 0, bedeuten und die Gruppe -Y-$R_2$ bevorzugt in 3- oder 4-Position steht, und von diesen Verbindungen vor allem diejenigen, in denen $R_1$ Wasserstoff, das Natriumkation, Methyl oder Aethyl, Y ein Sauerstoff- oder Schwefelatom, $R_2$ Trifluormethl, Difluormethyl, 1,1,2,2-Tetrafluoräthyl, 1,1,2,2,2-Pentafluoräthyl oder 1,2-Dichlorvinyl, $R_4$ Wasserstoff oder Methyl, $R_5$ Wasserstoff und n 0 bedeuten.

Besonders hervorzuheben sind Verbindungen der Formel I, in denen $R_1$ Wasserstoff, Methyl oder Aethyl, Y ein Sauerstoff- oder Schwefelatom, $R_2$ Trifluormethyl, Difluormethyl, 1,1,2,2-Tetrafluoräthyl oder 1,1,2,2,2-Pentafluoräthyl, $R_4$ Wasserstoff oder Methyl, $R_5$ Wasser-

stoff und n 0 bedeuten und die Gruppe $-Y-R_2$ bevorzugt in 4-Position steht, und von diesen Verbindungen insbesondere diejenigen, in denen $R_1$ für Wasserstoff, Methyl oder Aethyl, Y für ein Sauerstoffatom, $R_2$ für Trifluormethyl oder Difluormethyl, $R_4$ für Wasserstoff oder Methyl, $R_5$ für Wasserstoff und n für 0 steht und die Gruppe $-Y-R_2$ bevorzugt in 4-Position steht.

Bevorzugte Einzelverbindungen sind:
[1-(4-(1,1,2,2-Tetrafluoräthoxy)-phenyl)-1,4-dihydro-4-oxo-6-methyl-pyridazin-3-yl]-carbonsäure,
[1-(3-Methyl-4-(1,1,2,2-tetrafluoräthoxy)-phenyl)-1,4-dihydro-4-oxo-6-methyl-pyridazin-3-yl]-carbonsäure,
[1-(4-Trifluormethylthio)-phenyl)-1,4-dihydro-4-oxo-6-methyl-pyridazin-3-yl]-carbonsäure,
[1-(4-Difluormethylthio)-phenyl)-1,4-dihydro-4-oxo-6-methyl-pyridazin-3-yl]-carbonsäure,
[1-(4-(1,1,2,2,2-Pentafluoräthoxy)-phenyl)-1,4-dihydro-4-oxo-6-methyl-pyridazin-3-yl]-carbonsäure und insbesondere
[1-(4-(Trifluormethoxy)-phenyl)-1,4-dihydro-4-oxo-6-methyl-pyridazin-3-yl]-carbonsäure,
[1-(4-(Difluormethoxy)-phenyl)-1,4-dihydro-4-oxo-6-methyl-pyridazin-3-yl]-carbonsäure,
[1-(2-Methyl-4-(difluormethoxy)-phenyl)-1,4-dihydro-4-oxo-6-methyl-pyridazin-3-yl]-carbonsäure,
[1-(4-Trifluormethoxy)-phenyl)-1,4-dihydro-4-oxo-6-methyl-pyridazin-3-yl]-carbonsäure-methylester und
[1-(4-(Trifluormethoxy)-phenyl)-1,4-dihydro-4-oxo-6-methyl-pyridazin-3-yl]-carbonsäure-äthylester.

Die Herstellung von Verbindungen der Formel I erfolgt zweckmässigerweise, indem man ein Hydrazon der Formel V

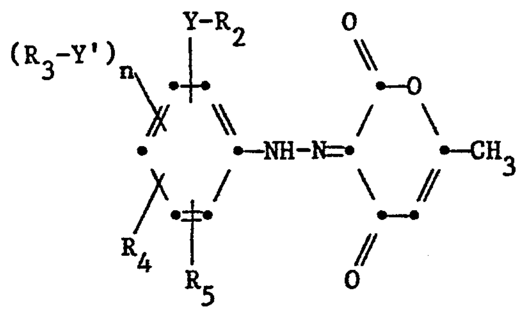 (V)

worin Y, Y', $R_2$, $R_3$, $R_4$, $R_5$ und n die für Formel I angegebenen Bedeutungen haben, durch Zugabe einer Säure, einer Base oder einem Amin zum Pyridazinylcarbonsäure-derivat der Formel I umlagert und gegebenenfalls, wenn $R_1$ in der Formel I für $C_1$-$C_6$-Alkyl steht, die erhaltene Verbindung in den entsprechenden Alkylester überführt.

Die Umlagerung eines Hydrazons der Formel V, worin Y, Y' $R_2$, $R_3$, $R_4$, $R_5$ und n die für Formel I angegebenen Bedeutungen haben, zu einem Pyridazinylcarbonsäure-derivat der Formel I, worin $R_1$ für $C_1$-$C_6$-Alkyl steht, kann zugleich mit einer Veresterung durchgeführt werden, indem man ein Hydrazon der Formel V mit einem Alkohol der Formel VI

$$R_1' - OH \qquad (VI)$$

worin $R_1'$ $C_1$-$C_6$-Alkyl bedeutet, unter den für die Umlagerung angegebenen Bedingungen umsetzt, wobei die Umsetzung bevorzugt in wasserfreiem Medium unter Einsatz eines Trocknungsmittels vorgenommen wird.

Die Herstellung von Hydrazonen der Formel V und ihre Umlagerung stellen ebenfalls einen Teil der Erfindung dar. Dabei wird das Verfahren zweckmässigerweise durchgeführt, indem man ein Anilin der Formel II

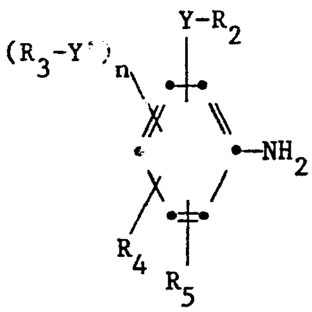

(II)

worin Y, Y', $R_2$, $R_3$, $R_4$, $R_5$ und n die für Formel I angegebenen Bedeutungen haben, in ein entsprechendes Diazoniumsalz der Formel III

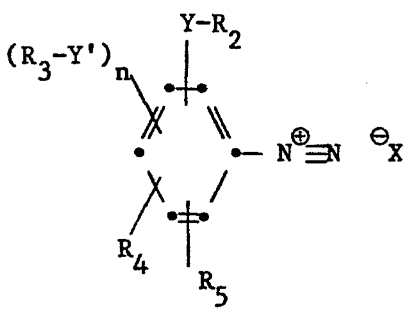

(III)

worin Y, Y', $R_2$, $R_3$, $R_4$, $R_5$ und n die für Formel I angegebenen Bedeutungen haben und X ein geeignetes Anion, beispielsweise ein Chloranion, bedeutet, überführt und dieses Diazoniumsalz mit dem Pyron der Formel IV

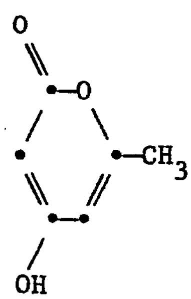

(IV)

oder einem Salz davon umsetzt und das erhaltene Hydrazon der Formel V

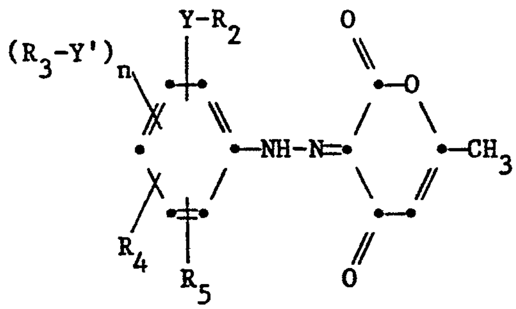

(V)

worin Y, Y', $R_2$, $R_3$, $R_4$, $R_5$ und n die für Formel I angegebenen Bedeutungen haben, durch Zugabe einer Säure, einer Base oder einem Amin, gegebenenfalls unter Umsetzung mit einem Alkohol der Formel VI

$$R_1'-OH \qquad (VI)$$

worin $R_1'$ $C_1$-$C_6$-Alkyl bedeutet, zum Pyridazinylcarbonsäurederivat der Formel I umlagert und gegebenenfalls, wenn die Umlagerung ohne Umsetzung mit einem Alkohol der Formel VI erfolgt und in der Formel I $R_1$ für $C_1$-$C_6$-Alkyl steht, die erhaltene Verbindung in den entsprechenden Alkylester überführt. Die Umsetzung eines Hydrazons der Formel V mit einem Alkohol der Formel VI erfolgt bevorzugt in wasserfreiem Medium in Gegenwart eines Trocknungsmittels.

Die Ueberführung des Anilins der Formel II in das entsprechende Diazoniumsalz der Formel III lässt sich auf an sich bekannte Weise durchführen, beispielsweise durch Umsetzung eines Anilins der Formel II mit konzentrierter Salzsäure und Natriumnitrit.

Die Umsetzung eines Diazoniumsalzes der Formel III mit einem Pyron der Formel IV erfolgt zweckmässigerweise in Gegenwart eines polaren Lösungsmittels. Als polare Lösungsmittel kommen beispielsweise niedere Alkohole wie Methanol, Aethanol oder Isopropanol, Dimethylformamid, 1,2-Dimethoxyäthan oder Wasser in Betracht.

Die Umsetzung erfolgt im allgemeinen in einem Temperaturbereich von -15° bis 60°C.

Die Umlagerung des erhaltenen Hydrazons der Formel V zum Pyridazinylcarbonsäure-derivat der Formel I lässt sich durch Zugabe einer Säure wie beispielsweise Schwefelsäure, Salzsäure, Salpetersäure, Bromwasserstoff, Phosphorsäure, Trifluoressigsäure, Methansulfonsäure oder Perchlorsäure, oder einer Base wie beispielsweise Natrium- oder Kaliumcarbonat oder Natrium- oder Kaliumhydroxyd, oder eines Amins wie beispielsweise Piperidin oder Morpholin, durchführen.

Die Temperatur liegt zweckmässigerweise im Bereich von 0° bis 150°C, bevorzugt 40° bis 120°C, und insbesondere 60° bis 100°C.

Die Umlagerung kann in einem inerten Lösungsmittel durchgeführt werden, wobei als Lösungsmittel beispielsweise halogenierte Kohlenwasserstoffe wie Chloroform, Tetrachlorkohlenstoff, Methylenchlorid oder Chlorbenzol, oder aromatische Kohlenwasserstoffe wie Benzol, Toluol oder Xylol verwendet werden können.

Bei der Veresterung eines Hydrazons der Formel V mit einem Alkohol der Formel VI kommen als Trocknungsmittel beispielsweise Phosphorpentoxyd, ein Tetra($C_1$-$C_4$-Alkyl)-silikat, ein Tri($C_1$-$C_4$-Alkyl)-borat, ein Tri($C_1$-$C_4$-Alkyl)-orthoformat, ein $C_1$-$C_4$-Alkylketal, ein $C_1$-$C_4$-Alkylacetal oder ein Molekularsieb wie beispielsweise ein Linde Typ 4-A Molekularsieb, in Betracht.

Die Ausgangsprodukte sind bekannt oder können analog bekannten Verfahren hergestellt werden.

Verschiedene Pyridazin-derivate mit pflanzenregulatorischer Wirkung sind in der amerikanischen Patentschrift 4.345.934 und den europäischen Patentpublikationen 25.498, 37.133 und 37.134 beschrieben worden.

Es wurde nunmehr gefunden, dass überraschenderweise die Pyridazinylcarbonsäure-derivate der Formel I und Mittel, die diese Wirkstoffe enthalten, sich vor allem dadurch auszeichnen, dass sie gezielt in

die Physiologie von Pflanzen eingreifen. Ein derartiger Eingriff in die physiologischen Vorgänge der Pflanzenentwicklung macht die Wirkstoffe der Formel I für verschiedene Zwecke verwendbar, insbesondere für solche, die mit der Ertragssteigerung von Nutzpflanzen und der Arbeitseinsparung bei der Züchtung und Produktion von Hybridsaatgut im Zusammenhang stehen.

Nach bisherigen Erfahrungen gilt für die Applikation von Wachstumsregulatoren, dass die Aktivsubstanzen eine oder mehrere unterschiedliche Wirkungen bei den Pflanzen hervorrufen können. Diese verschiedenartigen Wirkungen hängen im wesentlichen vom Zeitpunkt der Anwendung, d.h. vom physiologischen Zustand des Samens oder vom Entwicklungszustand der Pflanze, der Art der Applikation sowie insbesondere von den angewendeten Konzentrationen ab. Derartige Effekte sind wiederum je nach Pflanzenart verschieden. Die Applikation von Verbindungen der Formel I eröffnet nunmehr die Möglichkeit, das Wachstum von Pflanzen in gewünschter Weise zu beeinflussen.

Mit den Pyridazinylcarbonsäure-derivaten der Formel I bzw. mit agrochemischen Mitteln, die diese Verbindungen als Wirkstoff enthalten, lässt sich das generative Wachstum einer ganzen Reihe mono- und dicotyler Pflanzen steuern, wobei das vegetative Wachstum von Kulturpflanzen in weiten Konzentrationsbereichen positiv beeinflusst wird. Bei sehr hohen Aufwandmengen können phytotoxische Effekte auftreten. Der Einfluss auf das generative Pflanzenwachstum macht sich, je nach Pflanzenkultur, unterschiedlich bemerkbar.

Eine im Vordergrund stehende Art der Steuerung des generativen Wachstums beruht auf der besonderen Eigenschaft der hierin beschriebenen Pyridazinylcarbonsäure-derivate, bei verschiedenartigen Kulturpflanzen, insbesondere bei der Anwendung in Monocotyledonen, wie beispielsweise Weizen, Roggen, Gerste, Hafer, Reis, Mais, Sorghum-Hirse und Futtergräsern, aber auch bei anderen Pflanzen, wie beispielsweise Sonnenblumen, Tomaten, Lein, Leguminosen (Soja, Bohnen), Raps oder Baumwolle eine gametozide Wirkung zu entfalten.

Die Umlagerung des erhaltenen Hydrazons der Formel V zum Pyridazinylcarbonsäure-derivat der Formel I lässt sich durch Zugabe einer Säure wie beispielsweise Schwefelsäure, Salzsäure, Salpetersäure, Bromwasserstoff, Phosphorsäure, Trifluoressigsäure, Methansulfonsäure oder Perchlorsäure, oder einer Base wie beispielsweise Natrium- oder Kaliumcarbonat oder Natrium- oder Kaliumhydroxyd, oder eines Amins wie beispielsweise Piperidin oder Morpholin, durchführen.

Die Temperatur liegt zweckmässigerweise im Bereich von 0° bis 150°C, bevorzugt 40° bis 120°C, und insbesondere 60° bis 100°C.

Die Umlagerung kann in einem inerten Lösungsmittel durchgeführt werden, wobei als Lösungsmittel beispielsweise halogenierte Kohlenwasserstoffe wie Chloroform, Tetrachlorkohlenstoff, Methylenchlorid oder Chlorbenzol, oder aromatische Kohlenwasserstoffe wie Benzol, Toluol oder Xylol verwendet werden können.

Bei der Veresterung eines Hydrazons der Formel V mit einem Alkohol der Formel VI kommen als Trocknungsmittel beispielsweise Phosphorpentoxyd, ein Tetra($C_1$-$C_4$-Alkyl)-silikat, ein Tri($C_1$-$C_4$-Alkyl)-borat, ein Tri($C_1$-$C_4$-Alkyl)-orthoformat, ein $C_1$-$C_4$-Alkylketal, ein $C_1$-$C_4$-Alkylacetal oder ein Molekularsieb wie beispielsweise ein Linde Typ 4-A Molekularsieb, in Betracht.

Die Ausgangsprodukte sind bekannt oder können analog bekannten Verfahren hergestellt werden.

Verschiedene Pyridazin-derivate mit pflanzenregulatorischer Wirkung sind in der amerikanischen Patentschrift 4.345.934 und den europäischen Patentpublikationen 25.498, 37.133 und 37.134 beschrieben worden.

Es wurde nunmehr gefunden, dass überraschenderweise die Pyridazinylcarbonsäure-derivate der Formel I und Mittel, die diese Wirkstoffe enthalten, sich vor allem dadurch auszeichnen, dass sie gezielt in

die Physiologie von Pflanzen eingreifen. Ein derartiger Eingriff in die physiologischen Vorgänge der Pflanzenentwicklung macht die Wirkstoffe der Formel I für verschiedene Zwecke verwendbar, insbesondere für solche, die mit der Ertragssteigerung von Nutzpflanzen und der Arbeitseinsparung bei der Züchtung und Produktion von Hybridsaatgut im Zusammenhang stehen.

Nach bisherigen Erfahrungen gilt für die Applikation von Wachstumsregulatoren, dass die Aktivsubstanzen eine oder mehrere unterschiedliche Wirkungen bei den Pflanzen hervorrufen können. Diese verschiedenartigen Wirkungen hängen im wesentlichen vom Zeitpunkt der Anwendung, d.h. vom physiologischen Zustand des Samens oder vom Entwicklungszustand der Pflanze, der Art der Applikation sowie insbesondere von den angewendeten Konzentrationen ab. Derartige Effekte sind wiederum je nach Pflanzenart verschieden. Die Applikation von Verbindungen der Formel I eröffnet nunmehr die Möglichkeit, das Wachstum von Pflanzen in gewünschter Weise zu beeinflussen.

Mit den Pyridazinylcarbonsäure-derivaten der Formel I bzw. mit agrochemischen Mitteln, die diese Verbindungen als Wirkstoff enthalten, lässt sich das generative Wachstum einer ganzen Reihe mono- und dicotyler Pflanzen steuern, wobei das vegetative Wachstum von Kulturpflanzen in weiten Konzentrationsbereichen positiv beeinflusst wird. Bei sehr hohen Aufwandmengen können phytotoxische Effekte auftreten. Der Einfluss auf das generative Pflanzenwachstum macht sich, je nach Pflanzenkultur, unterschiedlich bemerkbar.

Eine im Vordergrund stehende Art der Steuerung des generativen Wachstums beruht auf der besonderen Eigenschaft der hierin beschriebenen Pyridazinylcarbonsäure-derivate, bei verschiedenartigen Kulturpflanzen, insbesondere bei der Anwendung in Monocotyledonen, wie beispielsweise Weizen, Roggen, Gerste, Hafer, Reis, Mais, Sorghum-Hirse und Futtergräsern, aber auch bei anderen Pflanzen, wie beispielsweise Sonnenblumen, Tomaten, Lein, Leguminosen (Soja, Bohnen), Raps oder Baumwolle eine gametozide Wirkung zu entfalten.

Diese führt zu einer männlichen Sterilität der Pflanze, ohne die Befruchtbarkeit der weiblichen Blütenteile merklich zu beeinflussen. Bei zahlreichen Kulturpflanzen ist gleichzeitig eine Zunahme der Blütenansätze und/oder die Ausbildung parthenokarper Früchte zu beobachten. Die männliche Sterilität zeigt sich entweder in einer aktuellen männlichen Sterilität, nämlich darin, dass die männlichen Blütenteile überhaupt nicht gebildet werden oder darin, dass die Blütenpollen steril sind, oder sie zeigt sich in einer funktionellen männlichen Sterilität, bei der die männlichen Blütenteile zwar noch gebildet werden, aber nicht in der Lage sind, eine Befruchtung herbeizuführen. Die Wirkstoffe der Formel I können aber auch Protogynie hervorrufen, d.h. es werden vorzeitig befruchtungsfähige weibliche Blütenteile ausgebildet oder das Heranwachsen der männlichen Blütenteile zeitlich so verzögert, dass eine Fremdbestäubung mit ausgewählten Blütenpollen durchgeführt werden kann.

Diese gametoziden Effekte lassen sich besonders vorteilhaft bei der Züchtung und Feldproduktion von Hybrid-Saatgut einsetzen. Hybrid-Saatgut spielt bei Nutzpflanzen, insbesondere solchen, die für die Ernährung von Bedeutung sind, und bei Zierpflanzen eine bedeutende Rolle. Hybride sind üblicherweise vitaler als reine Arten und liefern höhere Erträge als die ertragsreichste Elternsorte.

Bei der Hybridisierung kreuzt der Züchter zwei oder mehr sorgfältig ausgewählte Inzuchtlinien in einem experimentell ermittelten Schema und erhält auf diese Weise Hybridsaatgut, das Pflanzen mit gesteigerter Wüchsigkeit und Leistung hervorbringt.

Bei den einhäusigen synözischen Maispflanzen lässt sich eine Hybridisierung besonders einfach auf konventionelle Weise durchführen, da männliche und weibliche Blütenteile an unterschiedlichen Stellen der Pflanze ausgebildet werden (Dikline Blüten). Die Antheren, die Pollen liefern, bilden die Spitze der Maispflanze, während die kolbenförmige, weibliche Infloreszenz mit den Narbenfäden unterhalb der Pflanzenmitte ausgebildet wird. Um nun $F_1$-Hybride zu züchten werden üblicherweise alternierende Reihen von

Maispflanzen der Sorten oder homozygoten Linien AA und BB angepflanzt. Zur Sicherstellung, dass der AA-Mais keine Pollen bildet, werden die AA-Pflanzen rechtzeitig, vor der vollständigen Entwicklung der männlichen Blütenstände manuell oder maschinell entfahnt und anschliessend mit Pollen einer BB-Mais-Sorte bestäubt, wobei auf den AA-Pflanzen Saatgut eines AB-Hybrids ($F_1$) entsteht. Der hierzu erforderliche Arbeitsvorgang ist nicht nur zeitraubend und aufwendig, sondern führt zwangsläufig zu einer Verletzung der Pflanze und besonders bei maschineller Entmännlichung zu einer unerwünschten Ertragsdepression bei der als weiblicher Elternteil (Samenträger) dienenden Linie/Sorte.

Bei einhäusigen, synözischen Pflanzen wie Mais lässt sich die Hybridisierung nach der geschilderten, herkömmlichen Methode noch einigermassen wirtschaftlich bewerkstelligen. Wesentlich schwieriger gestaltet sich dieser Vorgang jedoch bei kleinkörnigen Halmfrüchten, insbesondere bei jenen, die Zwitterblüten aufweisen und bei denen normalerweise Selbstbestäubung (sog. Selbstbefruchtung), aber auch Kreuzbestäubung (sog. Fremdbefruchtung) auftritt. In diesen Fällen erweist sich das herkömmliche Verfahren als äusserst zeitraubend, arbeitsintensiv, unwirtschaftlich, und es bedarf vor allem speziell ausgebildeter Arbeitskräfte. Kleinkörnige Halmfrucht-Hybride lassen sich nur züchten, wenn die Selbst- und Kreuzbestäubung in der Mutterlinie vollständig unterdrückt wird. In der Praxis muss man dazu jede der winzigen Blüten frühzeitig von Hand öffnen, alle Staubbeutel sorgfältig entfernen und anschliessend die Blüten gegen unerwünschte Fremdbestäubung schützen.

Bei einigen Getreidesorten wie Weizen, Gerste sowie Mais und bei Dicotylen wird noch eine weitere Hybridisierungsmethode praktiziert. Bei diesem Verfahren geht man von cytoplasmatisch, männlich sterilen Pflanzen aus und führt Fremdbestäubung durch. Diese cytoplasmatisch sterilen Pflanzen beschränken sich oft auf Mutterlinien, die dasselbe Cytoplasma aufweisen. Dadurch werden cytoplasmatisch vererbte Schwächen oder Fehler, z.B. mangelnde Resistenz gegen einen bestimmten Krankheitserreger oder Frostempfindlichkeit und ähnliches, bei dieser Methode zwangsläufig auf alle, von dieser Mutterlinie abstammenden Hybride übertragen. Ausserdem erfordert die Hybridisierung mit cytoplasmatisch, männlich sterilen Linien, insbesondere bei kleinkörnigen Halmfrüchten, aber auch in dicotylen Kulturen aufwendige Massnahmen von erhöhtem Schwierigkeitsgrad.

Unabhängig von der Methode setzt die Züchtung von Hybrid-Saatgut stets die Erzeugung männlich steriler und weiblich intakter Pflanzen voraus. Eine einfache, praktikable und wirtschaftliche Lösung zur Herstellung einer selektiven männlichen Sterilität bietet der Einsatz von chemischen Sterilisierungsmitteln (Gametoziden). Pyridazinylcarbonsäure-derivate der Formel I zeigen sehr gute männlich-gametozide Eigenschaften und sind daher für diese und verwandte Zwecke geeignet. Ihr Einsatz in Kulturen von Nutzpflanzen lässt die meisten Probleme, die an herkömmliche Hybridisierungsmethoden gekoppelt sind, gar nicht erst in Erscheinung treten.

Zur Erzeugung von männlich sterilen Pflanzen und damit zur Gewinnung von Hybrid-Saatgut kann man im einzelnen folgendermassen vorgehen: Beide Elternpflanzen, die miteinander gekreuzt werden sollen, werden in z.B. alternierenden Reihen gepflanzt. Die als Samenträger oder Mutterpflanze ausgewählte Linie wird zu Beginn der Blütenbildung, aber noch vor Ausbildung der männlichen Blütenteile, mit einem der Wirkstoffe der Formel I behandelt. Auf diese Weise erhält man eine Reihe männnlich steriler, aber fruchtbarer weiblicher Elternpflanzen. Die andere Reihe bleibt unbehandelt und dient als Pollenspender. Ihre männlichen Blütenteile gelangen zur vollen Ausbildung und liefern die Pollen zur Bestäubung der Mutterpflanze bzw. des Samenträgers. Die von der Mutterpflanze erzeugten Samenkörner stellen das Hybridsaatgut dar und können auf konventionelle Weise geerntet werden. Die Körner der männlichen Elternpflanzen werden separat geerntet und anderweitig genutzt.

Das beschriebene Verfahren zur Erzeugung von männlich sterilen Pflanzen bzw. zur Gewinnung von Hybridsaatgut ist Bestandteil der vorliegenden Erfindung.

Darüberhinaus bewirken die Wirkstoffe der Formel I noch weitere wachstumsregulierende Effekte, beispielsweise eine zeitliche Steuerung der Blütenbildung und in der Folge ein kontrollierte Samen-oder Fruchtreifung. Diese Art der Blühstimulation ist insbesondere bei solchen Pflanzensorten von wirtschaftlichem Interesse, die gleichzeitig blühen und Früchte tragen. So führt beispielsweise die Behandlung von Avocado- oder Baumwollpflanzen mit Verbindungen der Formel I einerseits zu einer vorteilhaften Zunahme der Zahl der Blütenstände, andererseits wird der Blüh- und Reifeprozess einem kontrollierten Rhythmus unterworfen. Auf diese Weise kann nicht nur eine Ertragssteigerung erreicht werden, sondern eine rationellere Gestaltung der Ernte und somit eine günstigere Vermarktung.

Besonders erwähnenswert ist die Regulation von Blütenbildung und -ausgestaltung durch Behandlung kleinkörniger Halmfruchtpflanzen, von Getreidepflanzen oder Futtergräsern nach dem Auflaufen der Pflanzen, aber vor dem Sichtbarwerden von Aehren und Antheren, mit einer Verbindung der Formel I gemäss Anspruch 1. Als sehr günstig erweist sich die Behandlung solcher Pflanzen im 5 1/2-Blattstadium.

Eine weitere vorteilhafte Variante des erfindungsgemässen Verfahrens besteht in der Behandlung von Kulturpflanzen vor Blühbeginn, wobei als Kulturpflanzen besonders Sonnenblumen-, Baumwoll-, Kürbis-, Gurken-, Melonen-, Hülsenfrucht- oder Zierpflanzen zu erwähnen sind.

In manchen Fällen bewirkt die Applikation von Verbindungen der Formel I eine deutliche Verlängerung der Blühperiode, dadurch kann die Möglichkeit der Bestäubung aller Blüten erhöht werden. Eine zeitliche Dehnung der Blühphase ist auch bei einer ganzen Anzahl von Zierpflanzen, insbesondere bei Blumen wünschenswert.

Bei einer ganzen Reihe unterschiedlicher Kulturpflanzen beobachtet man nach deren Behandlung mit Wirkstoffen der Formel I parallel zur männlichen Sterilisierung einen positiven Einfluss auf die weiblichen Blütenstände. Auf diese Weise wird oftmals die Zahl der

weiblichen Blüten pro Blütenstand oder pro Pflanze und somit auch der Ertrag erhöht. Derartige Effekte sind häufig auch bei kleinkörnigen Halmfrüchten (wie Gerste), Gurkengewächsen, Sonnenblumen, Hülsenfrüchten (wie Soja), baumartigen Gewächsen und Zierpflanzen (wie Compositen) zu verzeichnen. In einigen Fällen treten weitere, verwandte wachstums-regulierende Wirkungen auf. Daneben kann mitunter bei gewissen Pflanzensorten auch eine verringerte Frostempfindlichkeit und eine erhöhte Resistenz gegenüber Krankheitserregern festgestellt werden.

Die vorliegende Erfindung betrifft somit auch die Verwendung der Pyridazinylcarbonsäure-derivate der Formel I oder von Mitteln, die diese Verbindungen als Aktivsubstanzen enthalten, zur Steuerung des Pflanzenwuchses, insbesondere zur Erzeugung einer Sterilität der männlichen Blütenteile (Einsatz als Gametozide) und/oder zur Förderung der weiblichen Blütenteile und aller daraus resultierender Sekundäreffekte wie z.B. Ertragssteigerung, Verlängerung der Blühdauer, vermehrte Blütenbildung, Steuerung der Fruchtbildung oder des Reifeprozesses und ähnliches.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet. Bestandteil dieser Erfindung sind auch alle neuen Wirkstoffe im Umfang der Formel I, einschliesslich ihres hierin beschriebenen Herstellungsverfahrens.

Zur Wuchsregulierung, insbesondere zur Erzielung einer gametoziden Wirkung, liegen die Aufwandmengen im allgemeinen pro Applikation zwischen 0,05 (bei Saatgutbehandlung auch niedriger) und 12, vorzugsweise 0,1 bis 4, insbesondere 0,1 bis 2 kg Aktivsubstanz pro ha. Als günstiger Applikationszeitpunkt, insbesondere bei Getreidekulturen, gilt die Periode nach dem Säen, aber noch bevor sich Aehren und Antheren zeigen bzw. das 5 1/2 Blattstadium oder die beginnende Blütenbildung.

Soll die Zahl der Blütenstände vermehrt oder die Blühdauer verlängert werden, z.B. bei Baumwolle, Sonnenblumen, Kürbis, Gurken, Melonen oder Zierpflanzen, so liegt eine vorteilhafte Dosierung bei 0,1 bis 4 kg AS/ha; Die Applikation erfolgt zweckmässigerweise in einem frühen Entwicklungsstadium der Pflanze.

Bei Pflanzen wie z.B. Soja, bei der Blüten und Früchte gleichzeitig auftreten, ist Splitapplikation angezeigt, d.h. die Applikation wird bevorzugt mit kleineren Aufwandmengen periodisch wiederholt. Im allgemeinen hängen die Aufwandmengen auch von der Applikationsart ab und liegen bei der einfachen Blattapplikation bevorzugt zwischen 0,1 bis 4 kg AS/ha, bei der Splitapplikation zwischen 0,1 bis 2 kg AS/ha und bei der Boden-(Drench)-applikation bei 0,1 bis 12 kg AS/ha, je nach Bodentyp.

In der Saatbeizung liegen die Aufwandmengen bei etwa 0,02 bis 1 kg AS pro 100 kg Saatgut.

Die beschriebenen Aufwandmengen und Applikationsarten sind ein Teil der vorliegenden Erfindung.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, gegebenenfalls zusammen mit weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Wird dieses Verfahren rechtzeitig zu Beginn der Blüte durchgeführt, so kann auf diese Weise eine sehr selektive männliche Sterilität erzeugt werden. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfalte, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes

Ferner kommen auch entsprechende Phosphate wie z.B. Salze des Phosphorsäureesters eines p-Nonylphenol-(4-14)-Aethylenoxyd-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt. Sehr gut geeignet im Rahmen der vorliegenden Erfindung ist beispielsweise eine 25%ige, wässrige Lösung von 4-tert.-Octylphenolpolglykoläther mit 8 Mol AeO (Extravon).

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood New Jersey, 1981
Stache, H., "Tensid-Taschenbuch",
Carl Hanser Verlag, München/Wien, 1981

Die agrochemischen Zubereitungen enthalten in der Regel 0,1 bis 99 %, insbesondere 0,1 bis 95 % Wirkstoff der Formel I, 99,9 bis 1 %, insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochmische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht.

Beispiel 1: Herstellung von [1-(2-Methyl-4-(difluormethoxy)-phenyl)-1,4-dihydro-4-oxo-6-methyl-pyridazin-3-yl]-carbonsäure

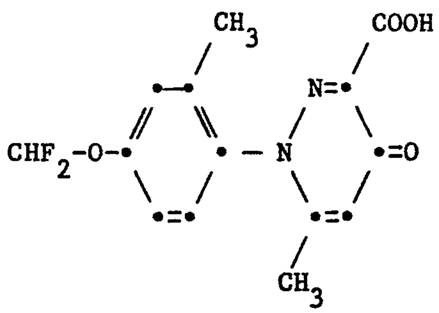

69,2 g 2-Methyl-4-(difluormethoxy)-anilin werden in 100 ml Wasser mit 160 ml konz. Salzsäure versetzt. Bei 0° bis 5°C lässt man unter kräftigem Rühren 56 ml einer 30 %igen Natriumnitrit-Lösung im Zeitraum von einer Stunde eintropfen. Die erhaltene klare Lösung lässt man bei 0° bis 5°C in eine Lösung von 51 g 4-Hydroxy-6-methyl-2-pyron und 220 g Natriumcarbonat in 1,5 Liter Wasser einfliessen. Es bildet sich ein dunkelgelber Niederschlag von Diazoniumion-Pyron-Kupplungsprodukt (Smp. 182-184°C). Das Gemisch wird noch einige Stunden bei Raumtemperatur gerührt, dann filtriert und mit Wasser gewaschen. Der feuchte Filterkuchen wird in einem Liter Wasser mit 100 g Soda bei 80°C bis zur vollständigen Lösung gerührt. Durch Ansäuern mit konz. Salzsäure wird die [1-(2-Methyl-4-(difluor-methoxy)-phenyl)-1,4-dihydro-4-oxo-6-methyl-pyridazin-3-yl]-carbonsäure ausgefällt. Nach Filtration, Trocknung und Umkristallisation aus Essigester/Hexan werden 91,4 g der obigen Carbonsäure erhalten. Smp. 155-156°C.

<u>Beispiel 2:</u> Herstellung von [1-(3-Methyl-4-(1,1,2,2-tetrafluor-äthoxy)-phenyl)-1,4-dihydro-4-oxo-6-methyl-pyridazin-3-yl]-carbon-säure

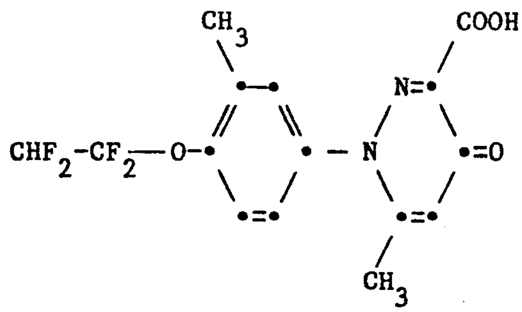

Analog wie im Beispiel 1 werden 66,9 g 3-Methyl-4-(1,1,2,2-tetrafluoräthoxy)-anilin in verdünnter Salzsäure mit Natriumnitrit diazotiert und die erhaltene Lösung mit 4-Hydroxy-6-methyl-2-pyron alkalisch gekuppelt. Das entstandene Kupplungsprodukt (Smp. 161-163°C) wird abfiltriert und noch feucht in einen Liter 20 %ige Salzsäure gegeben und das Gemisch unter Rühren am Rückfluss erhitzt. Nach etwa 4 Stunden ist die Reaktion beendet und man lässt abkühlen, filtriert, trocknet und kristallisiert aus Essigester/Hexan um. Man erhält 96,5 g [1-(3-Methyl-4-(1,1,2,2-(tetrafluoräthoxy)-phenyl)-1,4-dihydro-4-oxo-6-methyl-pyridazin-3-yl]-carbonsäure. Smp. 170°-172°C.

<u>Beispiel 3:</u> Herstellung des Natriumsalzes von [1-(3-Methyl-4-(1,1,2,2-tetrafluoräthoxy)-phenyl)-1,4-dihydro-4-oxo-6-methyl-pyridazin-3-yl]-carbonsäure
5 g [1-(3-Methyl-4-(1,1,2,2-(tetrafluoräthoxy)-phenyl)-1,4-dihydro-4-oxo-6-methyl-pyridazin-3-yl]-carbonsäure werden in 100 ml Methanol mit 2,5 g einer 30 %igen Natriummethylat-Lösung in Methanol versetzt und das Gemisch eingedampft. Der erhaltene halbfeste Rückstand wird in wenig Methylenchlorid aufgenommen und eingedampft. Man erhält das obige Nariumsalz (•1/2 $H_2O$). Smp. > 260°C.

<u>Beispiel 4:</u> Herstellung von [1-(3-Methyl-4-(1,1,2,2-tetrafluoräthoxy)-phenyl)-1,4-dihydro-4-oxo-6-methyl-pyridazin-3-yl]-carbonsäure-methylester
10 g [1-(3-Methyl-4-(1,1,2,2-tetrafluoräthoxy)-phenyl)-1,4-dihydro-4-oxo-6-methyl-pyridazin-3-yl]-carbonsäure werden in 50 ml Methanol mit 10 ml konz. Schwefelsäure unter Rühren versetzt. Man lässt die

Reaktionsmischung 24 Stunden stehen, giesst dann auf Eiswasser und nimmt das Produkt mit Chloroform auf. Die Chloroformlösung wird zweimal mit Sodalösung und anschliessend mit Wasser neutral gewaschen, getrocknet, eingeengt und durch Zugabe von Hexan zur Kristallisation gebracht. Man erhält 5,5 g des obigen Esters. Smp. 152-155°C.

Beispiel 5: Herstellung von [1-(4-(Trifluormethoxy)-phenyl)-1,4-dihydro-4-oxo-6-methyl-pyridazin-3-yl]-carbonsäure

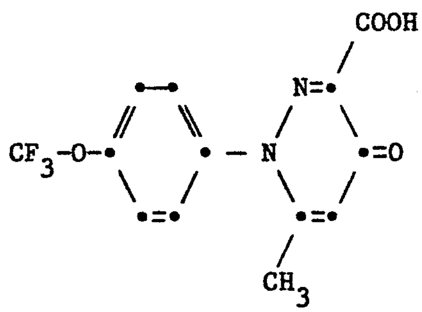

Zu 140 ml 32 %iger Salzsäure in 120 ml Wasser lässt man 53,1 g 4-Trifluormethoxy-anilin unter Rühren zufliessen und kühlt dann die entstandene Suspension ab. Bei -5° bis 0°C werden in 30 Minuten 10,5 g Natriumnitrit in 10,5 ml Wasser eingetropft. Nach 10 Minuten wird der Ueberschuss an Nitrit mit Sulfaminsäure entfernt. Die erhaltene Diazoniumlösung lässt man in 10 Minuten in eine Lösung von 37,8 g 4-Hydroxy-6-methyl-2-pyron und 170 g Natriumcarbonat in 1,5 Liter Wasser einfliessen. Nach 2 Stunden wird der Niederschlag abfiltriert, mit Wasser gewaschen und der noch feuchte Filterkuchen in 770 ml konz. Salzsäure 4 Stunden am Rückfluss erhitzt. Nach dem Abkühlen wird das Produkt abfiltriert, mit Wasser neutral gewaschen, getrocknet und aus Essigester/Hexan umkristallisiert. Man erhält 65 g [1-(4-(Trifluormethoxy)-phenyl)-1,4-dihydro-4-oxo-6-methyl-pyridazin-3-yl]-carbonsäure. Smp. 192-194°C.

Analog einer der vorstehend beschriebenen Methoden lassen sich auch die folgenden, in der Tabelle 1 zusammen mit den Verbindungen der vorstehenden Beispiele aufgeführten Verbindungen der Formel I herstellen:

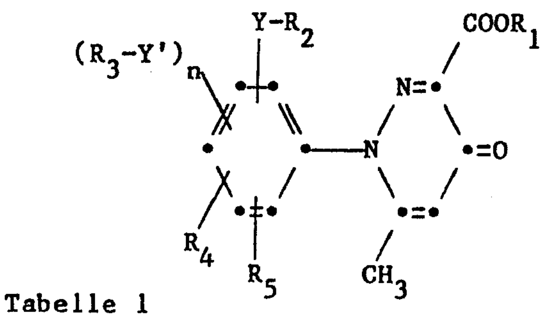

(I)

Tabelle 1

| Nr. | $R_1$ | Y | $R_2$ | Y' | $R_3$ | $R_4$ | $R_5$ | n | Smp.°C |
|---|---|---|---|---|---|---|---|---|---|
| 1 | H | 4-O | $CF_3$ | - | - | H | H | 0 | 192-194 |
| 2 | H | 4-O | $CHF_2$ | - | - | H | H | 0 | 161-163 |
| 3 | H | 4-O | $CF_2CHF_2$ | - | - | H | H | 0 | 175-177 |
| 4 | $Na^{\oplus}$ (•1/2 $H_2O$) | 4-O | $CF_2CHF_2$ | - | - | 3-$CH_3$ | H | 0 | > 260 |
| 5 | H | 4-O | $CF_2CHF_2$ | - | - | 3-$CH_3$ | H | 0 | 170-172 |
| 6 | $CH_3$ | 4-O | $CF_2CHF_2$ | - | - | 3-$CH_3$ | H | 0 | 152-155 |
| 7 | H | 3-O | $CF_2CHF_2$ | - | - | H | H | 0 | 178-180 |
| 8 | H | 4-O | $CHF_2$ | - | - | 2-$CH_3$ | H | 0 | 155-156 |
| 9 | $CH_3$ | 4-O | $CF_3$ | - | - | 2-Cl | H | 0 | |
| 10 | $C_2H_5$ | 4-O | $CHF_2$ | - | - | 2-Cl | H | 0 | |
| 11 | $CH_3$ | 3-O | $CHF_2$ | - | - | 4-Cl | H | 0 | |
| 12 | H | 4-O | $CHF_2$ | 2-O | $CHF_2$ | H | H | 1 | |

Tabelle 1 (Fortsetzung)

| Nr. | $R_1$ | Y | $R_2$ | Y' | $R_3$ | $R_4$ | $R_5$ | n | Smp.°C |
|---|---|---|---|---|---|---|---|---|---|
| 13 | H | 4-S | $CF_3$ | - | - | H | H | 0 | 182-183 |
| 14 | H | 4-S | $CHF_2$ | - | - | H | H | 0 | 212-213 (Z) |
| 15 | H | 4-O | $CF_2CF_3$ | - | - | H | H | 0 | 208-209 (Z) |
| 16 | H | $4-SO_2$ | $CF_3$ | - | - | H | H | 0 | |
| 17 | $CH_3$ | 4-O | $CF_3$ | - | - | 3-Cl | H | 0 | |
| 18 | $C_2H_5$ | 4-O | $CHF_2$ | - | - | $3-NO_2$ | H | 0 | |
| 19 | $CH_3$ | 3-O | $CF_2CHF_2$ | - | - | 4-Cl | H | 0 | |
| 20 | H | 3-O | $CF_3$ | - | - | H | H | 0 | 138-141 |
| 21 | H | 3-O | $CHF_2$ | - | - | H | H | 0 | |
| 22 | H | 3-S | $CF_3$ | - | - | H | H | 0 | |
| 23 | H | 4-O | $CF_3$ | - | - | 2-Cl | H | 0 | |
| 24 | $NH_4^{\oplus}$ | 4-O | $CHF_2$ | - | - | 2-Cl | H | 0 | |
| 25 | H | 3-O | $CHF_2$ | - | - | 4-Cl | H | 0 | |
| 26 | $C_3H_7n$ | 4-O | $CHF_2$ | 2-O | $CHF_2$ | H | H | 1 | |
| 27 | $C_3H_7iso$ | 4-S | $CF_3$ | - | - | H | H | 0 | |
| 28 | $C_3H_7iso$ | 4-S | $CHF_2$ | - | - | H | H | 0 | |
| 29 | $C_4H_9n$ | 4-O | $CF_2CF_3$ | - | - | H | H | 0 | |
| 30 | $Mg^{++}$ | $4-SO_2$ | $CF_3$ | - | - | H | H | 0 | |

Tabelle 1 (Fortsetzung)

| Nr. | $R_1$ | Y | $R_2$ | Y' | $R_3$ | $R_4$ | $R_5$ | n | Smp. °C |
|---|---|---|---|---|---|---|---|---|---|
| 31 | H | 4-O | $CF_3$ | - | - | 3-Cl | H | 0 | |
| 32 | $NH_4^{\oplus}$ | 4-O | $CHF_2$ | - | - | 3-$NO_2$ | H | 0 | |
| 33 | H | 3-O | $CF_2CHF_2$ | - | - | 4-Cl | H | 0 | |
| 34 | $C_3H_7n$ | 3-O | $CF_3$ | - | - | H | H | 0 | |
| 35 | $C_3H_7iso$ | 3-O | $CHF_2$ | - | - | H | H | 0 | |
| 36 | $C_4H_9n$ | 3-S | $CF_3$ | - | - | H | H | 0 | |
| 37 | $K^{\oplus}$ | 4-O | $CHF_2$ | 2-O | $CHF_2$ | H | H | 1 | |
| 38 | $K^{\oplus}$ | 3-O | $CF_3$ | - | - | H | H | 0 | |
| 39 | $Na^{\oplus}$ | 4-O | $CF_3$ | - | - | H | H | 0 | |
| 40 | $K^{\oplus}$ | 4-O | $CF_3$ | - | - | H | H | 0 | |
| 41 | $NH_4^{\oplus}$ | 4-O | $CF_3$ | - | - | H | H | 0 | |
| 42 | $CH_3$ | 4-O | $CF_3$ | - | - | H | H | 0 | 136-137 |
| 43 | $C_2H_5$ | 4-O | $CF_3$ | - | - | H | H | 0 | 129-130 |
| 44 | H | 4-O | $CCl=CCl_2$ | - | - | H | H | 0 | |
| 45 | H | 4-O | CCl=CHCl | - | - | H | H | 0 | 178-180 |

Formulierungsbeispiele für flüssige Wirkstoffe der Formel I
(% = Gewichtsprozent)

6. Emulsions-Konzentrat

| | |
|---|---|
| Wirkstoff aus Tabelle 1 | 25 % |
| Ca-Dodecylbenzolsulfonat | 5 % |
| Ricinusöl-polyäthylenglykoläther (36 Mol AeO) | 5 % |
| Tributylphenol-polyäthylenglykoläther (30 Mol AeO) | - |
| Cyclohexanon | - |
| Xylolgemisch | 65 % |

Aus solchen Konzentraten können durch Verdünnen mit Wasser Emulsionen jeder gewünschten Konzentration hergestellt werden.

| 7. Lösungen | a) | b) |
|---|---|---|
| Wirkstoff aus Tabelle 1 | 80 % | 10 % |
| Aethylenglykol-monomethyl-äther | 20 % | - |
| Polyäthylenglykol M G 400 | - | 70 % |
| N-Methyl-2-pyrrolidon | - | 20 % |

Die Lösungen sind zur Anwendung in Form kleinster Tropfen geeignet.

| 8. Granulate | a) | b) |
|---|---|---|
| Wirkstoff aus Tabelle 1 | 5 % | 10 % |
| Kaolin | 94 % | |
| Hochdisperse Kieselsäure | 1 % | |
| Attapulgit | - | 90 % |

Der Wirkstoff wird in Methylenchlorid gelöst, auf den Träger aufgesprüht und das Lösungsmittel anschliessend im Vakuum abgedampft.

| 9. Stäubemittel | a) | b) |
|---|---|---|
| Wirkstoff aus Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemitel.

Formulierungsbeispiele für feste Wirkstoffe der Formel I
(% = Gewichtsprozent)

| 10. Spritzpulver | a) | b) | c) |
|---|---|---|---|
| Wirkstoff aus Tabelle 1 | 25 % | 50 % | 75 % |
| Na-Ligninsulfonat | 5 % | 5% | - |
| Na-Laurylsulfat | 3 % | - | 5% |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 11. Emulsions-Konzentrat | |
|---|---|
| Wirkstoff aus Tabelle 1 | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentation hergestellt werden.

| 12. Stäubemittel | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| 13. Extruder Granulat | |
|---|---|
| Wirkstoff aus Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 14. Umhüllungs-Granulat | |
|---|---|
| Wirkstoff aus Tabelle 1 | 3 % |
| Polyäthylenglykol (M G 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 15. Suspensions-Konzentrat | |
|---|---|
| Wirkstoff aus Tabelle 1 | 40 % |
| Aethylenglykol | 10 % |
| Nonylphenolpolyethylenglykolether (15 Mol AeO) | 6 % |
| Na-Ligninsulfonat | 10 % |
| Carboxymethylcellulose | 1 % |

| | |
|---|---|
| 37-%ige wässrige Formaldehyd-Lösung | 0,2 % |
| Silikonöl in Form einer 75-%igen wässrigen Emulsion | 0,8 % |
| Wasser | 32 % |

Der feingemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können. Formulierungszusätze, die die Haftung des Wirkstoffs auf der Pflanze erhöhen, wie z.B. mineralische oder pflanzliche Oele, wirken sich bei der Blattapplikation sehr vorteilhaft aus.

Biologische Beispiele:

Beispiel 16: Gametozide Aktivität bei kleinkörnigen Halmfrüchten

a) Induktion der männlichen Sterilität

Sommerweizen wird in Töpfen von 18 x 18 x 18 cm in Kompost-haltiger und mit Dünger angereicherten Erde angezogen. Die Anzucht erfolgt bei 12 bis 15°C und im Kurztag (10 h).

Nach der Bestockung wird die Temperatur auf 18 bis 24°C erhöht und die Tageslänge auf 14 h erweitert. Die jungen Pflanzen werden präventiv oder kurativ gegen Blattläuse und Mehltau gespritzt. Im Moment, wo das Fahnenblatt erscheint (Stadium 7 nach der Skala von Feekes), werden dann die Versuchssubstanzen gespritzt. Die Spritzbrühe enthält 2000 oder 500 ppm Testsubstanz sowie 0,1 % Extravon und wird in einer Aufwandmenge, welche 800 l/ha entspricht, appliziert.

Nach der Applikation, jedoch vor Erscheinen der Antheren wird jede Aehre durch Abdecken gegen Fremdbestäubung geschützt. Die Bewertung der Gametozid-Wirkung erfolgt in der Reifung durch Auszählen der gebildeten Körner pro Aehre. Als Vergleich dienen unbehandelte Weizenpflanzen, deren Aehren ebenfalls abgedeckt worden sind.

In der folgenden Tabelle sind einige Resultate aus dem Test a) dargestellt:

Tabelle 2: Gametozide Aktivität (%)

| Konzentration, ppm | Verbindung Nr. 3 | 4 | 5 | 6 | 7 |
|---|---|---|---|---|---|
| 500 | 100 | 100 | 83,7 | 45,9 | 12,2 |
| 2000 | 100 | 100 | 100 | 100 | 24,6 |

Entsprechende Ergebnisse sind auch bei analogen Versuchen mit Gerste und Roggen zu beobachten.

b) Fruchtbarkeitstest (Bildung von Hybridsaatgut)

Eine Kontrollgruppe von Weizenpflanzen wird wie unter a) behandelt, abgedeckt oder isoliert gehalten und mit Blütenpollen einer anderen Weizensorte fremdbestäubt. Die Bewertung der Befruchtungsfähigkeit erfolgt zum Erntezeitpunkt durch Auszählen der gebildeten Hybrid-Körner pro Aehre. Als Vergleich dienen unbehandelte Weizenpflanzen und behandelte, aber abgedeckte Pflanzen.

Im Test b) zeigt sich, dass die Befruchtungsfähigkeit der im Test a) verwendeten Pflanzen vollständig oder weitgehend erhalten bleibt.

Beispiel 17: Gametozide Aktivität bei Weizen

Sommerweizen der Sorte "Sonora" wird in Töpfen von 18 x 18 x 18 cm in Kompost-haltiger und mit Dünger angereicherter Erde angezogen. Die Anzucht erfolgt bei 12 bis 15°C und im Kurztag (10 h).

Nach der Bestockung wird die Temperatur auf 18 bis 24°C erhöht und die Tageslänge auf 14 h erweitert. Die jungen Pflanzen werden präventiv oder kurativ gegen Blattläuse und Mehltau gespritzt.

Wenn die Aehrenprimordien am Haupttrieb eine Länge von 2 bis 3 mm (Frühapplikation) oder eine Länge von 8 bis 10 mm (Spätapplikation) erreicht haben, werden die Pflanzen mit einer aus einer 10%igen wässrigen Lösung von [1-(4-(Trifluormethoxy)-phenyl)-1,4-dihydro-4-oxo-6-methyl-pyridazin-3-yl]-carbonsäure hergestellten Spritzbrühe in einer Aufwandmenge, welche 13, 40 und 120 g Wirkstoff/ha entspricht, gleichmässig auf die Pflanzen gespritzt. Die als Spritzbrühe eingesetzte Lösung enthält 16,25 ppm, 50 ppm und 150 ppm. Die Spritzbrühe wird in einer Aufwandmenge, welche 800 l/ha entspricht, appliziert.

Nach der Applikation, jedoch vor Erscheinen der Antheren wird bei der Hälfte der Pflanzen jede Aehre durch Abdecken gegen Fremdbestäubung geschützt. Die Bewertung der Gametozid-Wirkung erfolgt in der Reifung durch Auszählen der gebildeten Körner pro Aehre. Als Vergleich dienen unbehandelte, abgedeckte und nicht abgedeckte Weizenpflanzen (Kontrolle = 100 %). Die Ergebnisse sind in der folgenden Tabelle dargestellt.

Tabelle 3: Körner pro Aehre in % der Kontrolle

| Anwendung | abgedeckt | | | nicht abgedeckt | | |
|---|---|---|---|---|---|---|
| | Aufwandmenge g AS/ha | | | Aufwandmenge g AS/ha | | |
| | 13 | 40 | 120 | 13 | 40 | 120 |
| Frühapplikation | 95 | 0 | 0 | 100 | 69 | 50 |
| Spätapplikation | 0 | 0 | 0 | 49 | 41 | 11 |

Beispiel 18: Gametozide Aktivität bei Weizen (systemisch)

Sommerweizen der Sorte "Calanda" wird, wenn die Aehrenprimordien am Haupttrieb eine Länge von 2 bis 3 mm erreicht haben, mit einer aus einer 10%igen wässrigen Lösung hergestellten Giessbrühe mit einem Wirkstoffgehalt von 180 und 540 ppm so gegossen, dass in den Pflanzenreihen 100 ml Lösung /Laufmeter gleichmässig appliziert werden. Die Aufwandmenge an Wirkstoff beträgt 1 kg/ha und 3 kg/ha.

Nach der Applikation, jedoch vor Erscheinen der Antheren wird bei der Hälfte der Pflanzen jede Aehre durch Abdecken gegen Fremdbestäubung geschützt. Die Bewertung der Gametozid-Wirkung erfolgt in der Reifung durch Auszählen der gebildeten Körner pro Aehre. Als Vergleich dienen unbehandelte, abgedeckte und nicht abgedeckte Weizenpflanzen (Kontrolle = 100 %). Die Ergebnisse zeigt die folgende Tabelle.

Tabelle 4: Körner pro Aehre in % der Kontrolle

| Verbindung Nr. | abgedeckt | | nicht abgedeckt | |
|---|---|---|---|---|
| | Aufwandmenge kg AS/ha | | Aufwandmenge kg AS/ha | |
| | 1 | 3 | 1 | 3 |
| 1 | 6.3 | 0 | 34.4 | 16.0 |
| 2 | 64.7 | 4.4 | 65.8 | 27.8 |

Beispiel 19: Gametozide Aktivität bei Soja (systemisch)

Sojapflanzen der Sorte "MAPLE ARROW" werden nach dem Erscheinen der ersten Blütenknospen mit einer aus einer 10%igen wässrigen Wirkstofflösung hergestellten Giessbrühe mit einem Wirkstoffgehalt von 25 ppm, 75 ppm und 225 ppm so gegossen, dass in den Pflanzenreihen 1 Liter Brühe pro 1,5 Laufmeter gleichmässig appliziert wird, wobei eine Benetzung der Blätter sorgfältig vermieden wird. Die Aufwandmenge an Wirkstoff beträgt 0,5 kg/ha, 1,5 kg/ha und 4,5 kg/ha. Die

Bewertung der Gametozid-Wirkung erfolgt zum Erntezeitpunkt nach folgenden Kriterien: Anzahl junger grüner Schoten pro Parzelle, Anzahl normaler Schoten pro Parzelle und Anzahl Samen pro Schote. Zum Vergleich dienen unbehandelte Sojapflanzen (Kontrolle). Die Ergebnisse sind in der nachfolgenden Tabelle dargestellt.

Tabelle 5:

Anzahl junger grüner Schoten/Parzelle (1,5 $m^2$) = gS/P
Anzahl normaler Schoten/Parzelle (1,5 $m^2$) = nS/P
Anzahl Samen/Schote = Sa/S

| | Verbindung Nr. | Aufwandmenge in kg AS/ha 0,5 | 1,5 | 4,5 | 0 (Kontrolle) |
|---|---|---|---|---|---|
| gS/P | 1 | 19 | 27 | 222 | 28 |
| gS/P | 2 | K | 8 | 16 | 28 |
| gS/P | 8 | 15 | 120 | 795 | 28 |
| nS/P | 1 | 629 | 799 | 530 | 814 |
| nS/P | 2 | K | 659 | 540 | 814 |
| nS/P | 8 | 785 | 1007 | 1068 | 814 |
| Sa/S | 1 | 2,24 | 2.13 | 1,90 | 2,11 |
| Sa/S | 2 | K | 2,07 | 1,86 | 2,11 |
| Sa/S | 8 | 2,10 | 1,48 | 0,24 | 2,11 |

K = wie unbehandelte Kontrolle

Beispiel 20: Gametozide Aktivität bei Soja

Sojapflanzen der Sorte "MAPLE ARROW" werden ein erstes Mal nach dem Erscheinen der ersten Blütenknospen und ein zweites Mal 4 Wochen nach der ersten Applikation mit einer aus einer 10%igen wässrigen

Wirkstofflösung hergestellten Spritzbrühe mit einem Wirkstoffgehalt von 500, 1 500 und 4 500 ppm gleichmässig besprüht. Die Aufwandmenge an Wirkstoff beträgt 0,2 kg/ha, 0,6 kg/ha und 1,8 kg/ha. Die Auswertung erfolgt zum Erntezeitpunkt. Die Ergebnisse sind in der nachfolgenden Tabelle dargestellt, wobei für die Werte "Anzahl Samen/Schote" nur normal ausgewachsene Schoten und Samen berücksichtigt worden sind.

Tabelle 6:

Anzahl junger grüner Schoten/Parzelle (1,5 $m^2$) = gS/P
Anzahl normaler Schoten/Parzelle (1,5 $m^2$) = nS/P
Anzahl Samen/Schote = Sa/S

| | Verbindung Nr. | Aufwandmenge in kg AS/ha 0,2 | 0,6 | 1,8 | 0 (Kontrolle) |
|---|---|---|---|---|---|
| gS/P | 8 | 27 | 38 | 385 | 31 |
| nS/P | 8 | 709 | 811 | 1'548 | 905 |
| Sa/S | 8 | 2,09 | 1,85 | 0,54 | 2,06 |

K = wie unbehandelte Kontrolle

Beispiel 21: Gametozide Wirkung bei Soja

Sojapflanzen der Sorte "MAPLE ARROW" werden wie in den Beispielen 19 und 20 mit einer Lösung der Testsubstanz in den dort angegebenen Anwendungskonzentrationen behandelt. Im Reifezustand wird die Anzahl der Samen pro Parzelle (1,5 $m^2$) in Prozent der Kontrolle (Kontrolle 100 %) ermittelt. Die Resultate sind in den folgenden Tabellen dargestellt.

Tabelle 7:
Versuchsanordnung wie Beispiel 19

| Verbindung Nr. | Aufwandmenge in kg AS/ha 0,5 | 1,5 | 4,5 |
|---|---|---|---|
| 1 | 82 | 99 | 59 |
| 2 | -- | 79 | 58 |
| 8 | 96 | 87 | 14 |

--: keine Bewertung

Tabelle 8:

Versuchsanordnung wie Beispiel 20

| Verbindung Nr. | Aufwandmenge in kg AS/ha 0,2 | 0,6 | 1,8 |
|---|---|---|---|
| 1 | 100 | 90 | 79 |
| 8 | 79 | 80 | 45 |

Beispiel 22: Gametozide Aktivität bei Weizen

Sommerweizen der Sorte "Sonora" wird in Töpfen von 18 x 18 x 18 cm in Kompost-haltiger und mit Dünger angereicherter Erde angezogen. Die Anzucht erfolgt bei 12 bis 15°C und im Kurztag (10 h).

Nach der Bestockung wird die Temperatur auf 18 bis 24°C erhöht und die Tageslänge auf 14 h erweitert. Die jungen Pflanzen werden präventiv oder kurativ gegen Blattläuse und Mehltau gespritzt.

Wenn die Aehrenprimordien am Haupttrieb eine Länge von 2 bis 3 mm (Frühapplikation) oder eine Länge von 8 bis 10 mm (Spätapplikation) erreicht haben, werden die Pflanzen mit einer aus Spritzpulver

des Wirkstoffs hergestellten Spritzbrühe in einer Aufwandmenge, welche 13 und 40 g Wirkstoff/ha entspricht, gleichmässig auf die Pflanzen gespritzt. Die als Spritzbrühe eingesetzte Suspension enthält 16,25 ppm und 50 ppm. Die Spritzbrühe wird in einer Aufwandmenge, welche 800 l/ha entspricht, appliziert.

Nach der Applikation, jedoch vor Erscheinen der Antheren wird jede Aehre durch Abdecken gegen Fremdbestäubung geschützt. Die Bewertung der Gametozid-Wirkung erfolgt in der Reifung durch Auszählen der gebildeten Körner pro Aehre. Als Vergleich dienen unbehandelte, abgedeckte Weizenpflanzen (Kontrolle = 100 %). Die Ergebnisse sind in der folgenden Tabelle dargestellt.

Tabelle 9: Körner pro Aehre in % der Kontrolle

| Anwendung | Verbindung Nr. | Aufwandmenge g AS/ha 13 | 40 |
|---|---|---|---|
| Frühapplikation | 42 | 0 | - |
| Spätapplikation | 42 | 0 | - |
| Frühapplikation | 43 | 0 | 0 |
| Spätapplikation | 43 | 0 | - |

- = keine Bewertung

Patentansprüche für die Vertragsstaaten: BE, DE, FR, IT, NL, SE, CH, LI

1. Pyridazinylcarbonsäure-derivate der Formel I

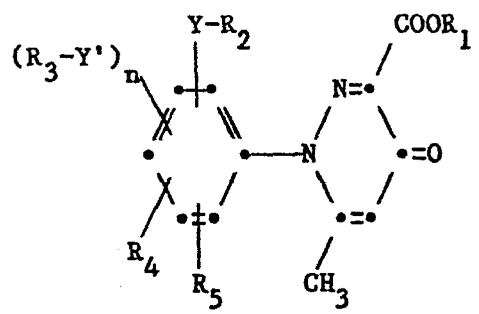

(I)

worin

$R_1$ Wasserstoff, ein Kation oder $C_1$-$C_6$-Alkyl,
Y und Y' unabhängig voneinander ein Sauerstoff- oder Schwefelatom oder eine Sulfinyl- oder Sulfonylgruppe,
$R_2$ und $R_3$ unabhängig voneinander eine ein- oder mehrfach durch Halogen substituierte $C_1$-$C_{12}$-Alkyl- oder $C_1$-$C_{12}$-Alkenylgruppe,
$R_4$ und $R_5$ unabhängig voneinander Wasserstoff, Halogen, Nitro, Cyan, $C_1$-$C_6$-Alkoxy oder unsubstituiertes oder ein- oder mehrfach durch Halogen substituiertes $C_1$-$C_6$-Alkyl und n 0 oder 1 bedeutet.

2. Pyridazinylcarbonsäure-derivate nach Anspruch 1, worin $R_1$ Wasserstoff, ein Alkalimetall- oder Erdalkalimetallkation, das Ammoniumkation oder $C_1$-$C_4$-Alkyl, Y und Y' unabhängig voneinander ein Sauerstoff- oder Schwefelatom oder eine Sulfonylgruppe, $R_2$ und $R_3$ unabhängig voneinander eine durch mindestens zwei Fluoratome substituierte $C_1$-$C_3$-Alkylgruppe oder eine durch mindestens zwei Chloratome substituierte $C_2$-$C_4$-Alkenylgruppe, $R_4$ Wasserstoff, Halogen, Nitro oder $C_1$-$C_4$-Alkyl, $R_5$ Wasserstoff und n 0 oder 1 bedeuten.

3. Pyridazinylcarbonsäure-derivate nach Anspruch 2, worin $R_1$ Wasserstoff, das Natrium-, Kalium-, Magnesium- oder Ammoniumkation oder $C_1$-$C_4$-Alkyl, Y ein Sauerstoff- oder Schwefelatom oder eine Sulfonylgruppe, $R_2$ Trifluormethyl, Difluormethyl, 1,1,2,2,2-Pentafluoräthyl, 1,1,2,2-Tetrafluoräthyl, 1,2-Dichlorvinyl oder 1,2,2-Trichlorvinyl, Y' ein Sauerstoffatom, $R_3$ Difluormethyl, $R_4$ Wasserstoff, Chlor, Nitro oder Methyl, $R_5$ Wasserstoff und n 0 oder 1 bedeuten.

4. Pyridazinylcarbonsäure-derivate nach Anspruch 3, worin $R_1$ Wasserstoff, das Natriumkation, Methyl oder Aethyl, Y ein Sauerstoff- oder Schwefelatom, $R_2$ Trifluormethyl, Difluormethyl, 1,1,2,2-Tetrafluoräthyl, 1,1,2,2,2-Pentafluoräthyl oder 1,2-Dichlorvinyl, $R_4$ Wasserstoff oder Methyl, $R_5$ Wasserstoff und n 0 bedeuten.

5. Pyridazinylcarbonsäure-derivate nach Anspruch 4, worin $R_1$ Wasserstoff, Methyl oder Aethyl, Y ein Sauerstoff- oder Schwefelatom, $R_2$ Trifluormethyl, Difluormethyl, 1,1,2,2-Tetrafluoräthyl oder 1,1,2,2,2-Pentafluoräthyl, $R_4$ Wasserstoff oder Methyl, $R_5$ Wasserstoff und n 0 bedeuten.

6. Pyridazinylcarbonsäure-derivate nach Anspruch 5, worin $R_1$ für Wasserstoff, Methyl oder Aethyl, Y für ein Sauerstoffatom, $R_2$ für Trifluormethyl oder Difluormethyl, $R_4$ für Wasserstoff oder Methyl, $R_5$ für Wasserstoff und n für 0 steht.

7. Pyridazinylcarbonsäure-derivat nach Anspruch 5, ausgewählt aus der Gruppe

[1-(4-(1,1,2,2-Tetrafluoräthoxy)-phenyl)-1,4-dihydro-4-oxo-6-methyl-pyridazin-3-yl]-carbonsäure,

[1-(3-Methyl-4-(1,1,2,2-tetrafluoräthoxy)-phenyl)-1,4-dihydro-4-oxo-6-methyl-pyridazin-3-yl]-carbonsäure,

[1-(4-Trifluormethylthio)-phenyl)-1,4-dihydro-4-oxo-6-methyl-pyridazin-3-yl]-carbonsäure,

[1-(4-Difluormethylthio)-phenyl)-1,4-dihydro-4-oxo-6-methyl-pyridazin-3-yl]-carbonsäure,

[1-(4-(1,1,2,2,2-Pentafluoräthoxy)-phenyl)-1,4-dihydro-4-oxo-6-methyl-pyridazin-3-yl]-carbonsäure,

[1-(4-(Trifluormethoxy)-phenyl)-1,4-dihydro-4-oxo-6-methyl-pyridazin-3-yl]-carbonsäure,

Patentansprüche für die Vertragsstaaten: BE, DE, FR, IT, NL, SE, CH, LI

1. Pyridazinylcarbonsäure-derivate der Formel I

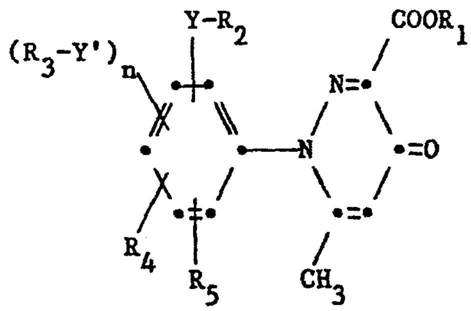

(I)

worin

$R_1$ Wasserstoff, ein Kation oder $C_1$-$C_6$-Alkyl,
Y und Y' unabhängig voneinander ein Sauerstoff- oder Schwefelatom oder eine Sulfinyl- oder Sulfonylgruppe,
$R_2$ und $R_3$ unabhängig voneinander eine ein- oder mehrfach durch Halogen substituierte $C_1$-$C_{12}$-Alkyl- oder $C_1$-$C_{12}$-Alkenylgruppe,
$R_4$ und $R_5$ unabhängig voneinander Wasserstoff, Halogen, Nitro, Cyan, $C_1$-$C_6$-Alkoxy oder unsubstituiertes oder ein- oder mehrfach durch Halogen substituiertes $C_1$-$C_6$-Alkyl und n 0 oder 1 bedeutet.

2. Pyridazinylcarbonsäure-derivate nach Anspruch 1, worin $R_1$ Wasserstoff, ein Alkalimetall- oder Erdalkalimetallkation, das Ammoniumkation oder $C_1$-$C_4$-Alkyl, Y und Y' unabhängig voneinander ein Sauerstoff- oder Schwefelatom oder eine Sulfonylgruppe, $R_2$ und $R_3$ unabhängig voneinander eine durch mindestens zwei Fluoratome substituierte $C_1$-$C_3$-Alkylgruppe oder eine durch mindestens zwei Chloratome substituierte $C_2$-$C_4$-Alkenylgruppe, $R_4$ Wasserstoff, Halogen, Nitro oder $C_1$-$C_4$-Alkyl, $R_5$ Wasserstoff und n 0 oder 1 bedeuten.

3. Pyridazinylcarbonsäure-derivate nach Anspruch 2, worin $R_1$ Wasserstoff, das Natrium-, Kalium-, Magnesium- oder Ammoniumkation oder $C_1$-$C_4$-Alkyl, Y ein Sauerstoff- oder Schwefelatom oder eine Sulfonylgruppe, $R_2$ Trifluormethyl, Difluormethyl, 1,1,2,2,2-Pentafluoräthyl, 1,1,2,2-Tetrafluoräthyl, 1,2-Dichlorvinyl oder 1,2,2-Trichlorvinyl, Y' ein Sauerstoffatom, $R_3$ Difluormethyl, $R_4$ Wasserstoff, Chlor, Nitro oder Methyl, $R_5$ Wasserstoff und n 0 oder 1 bedeuten.

4. Pyridazinylcarbonsäure-derivate nach Anspruch 3, worin $R_1$ Wasserstoff, das Natriumkation, Methyl oder Aethyl, Y ein Sauerstoff- oder Schwefelatom, $R_2$ Trifluormethyl, Difluormethyl, 1,1,2,2-Tetrafluoräthyl, 1,1,2,2,2-Pentafluoräthyl oder 1,2-Dichlorvinyl, $R_4$ Wasserstoff oder Methyl, $R_5$ Wasserstoff und n 0 bedeuten.

5. Pyridazinylcarbonsäure-derivate nach Anspruch 4, worin $R_1$ Wasserstoff, Methyl oder Aethyl, Y ein Sauerstoff- oder Schwefelatom, $R_2$ Trifluormethyl, Difluormethyl, 1,1,2,2-Tetrafluoräthyl oder 1,1,2,2,2-Pentafluoräthyl, $R_4$ Wasserstoff oder Methyl, $R_5$ Wasserstoff und n 0 bedeuten.

6. Pyridazinylcarbonsäure-derivate nach Anspruch 5, worin $R_1$ für Wasserstoff, Methyl oder Aethyl, Y für ein Sauerstoffatom, $R_2$ für Trifluormethyl oder Difluormethyl, $R_4$ für Wasserstoff oder Methyl, $R_5$ für Wasserstoff und n für 0 steht.

7. Pyridazinylcarbonsäure-derivat nach Anspruch 5, ausgewählt aus der Gruppe

[1-(4-(1,1,2,2-Tetrafluoräthoxy)-phenyl)-1,4-dihydro-4-oxo-6-methyl-pyridazin-3-yl]-carbonsäure,

[1-(3-Methyl-4-(1,1,2,2-tetrafluoräthoxy)-phenyl)-1,4-dihydro-4-oxo-6-methyl-pyridazin-3-yl]-carbonsäure,

[1-(4-Trifluormethylthio)-phenyl)-1,4-dihydro-4-oxo-6-methyl-pyridazin-3-yl]-carbonsäure,

[1-(4-Difluormethylthio)-phenyl)-1,4-dihydro-4-oxo-6-methyl-pyridazin-3-yl]-carbonsäure,

[1-(4-(1,1,2,2,2-Pentafluoräthoxy)-phenyl)-1,4-dihydro-4-oxo-6-methyl-pyridazin-3-yl]-carbonsäure,

[1-(4-(Trifluormethoxy)-phenyl)-1,4-dihydro-4-oxo-6-methyl-pyridazin-3-yl]-carbonsäure,

[1-(4-(Difluormethoxy)-phenyl)-1,4-dihydro-4-oxo-6-methyl-pyridazin-3-yl]-carbonsäure,
[1-(2-Methyl-4-(difluormethoxy)-phenyl)-1,4-dihydro-4-oxo-6-methyl-pyridazin-3-yl]-carbonsäure,
[1-(4-Trifluormethoxy)-phenyl)-1,4-dihydro-4-oxo-6-methyl-pyridazin-3-yl]-carbonsäure-methylester und
[1-(4-(Trifluormethoxy)-phenyl)-1,4-dihydro-4-oxo-6-methyl-pyridazin-3-yl]-carbonsäure-äthylester.

8. Verfahren zur Herstellung von Pyridazinylcarbonsäure-derivaten der Formel I gemäss Anspruch 1, dadurch gekennzeichnet, dass man ein Hydrazon der Formel V

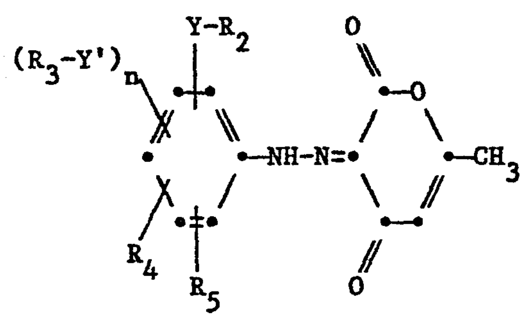 (V)

worin Y, Y', $R_2$, $R_3$, $R_4$, $R_5$ und n die für Formel I angegebenen Bedeutungen haben, durch Zugabe einer Säure, einer Base oder einem Amin zum Pyridazinylcarbonsäure-derivat der Formel I umlagert und gegebenenfalls, wenn $R_1$ in der Formel I für $C_1$-$C_6$-Alkyl steht, die erhaltene Verbindung in den entsprechenden Alkylester überführt.

9. Verfahren nach Anspruch 8, dadurch gekennzeichnet, dass man ein Hydrazon der Formel V mit einem Alkohol der Formel VI

$$R_1' - OH \qquad (VI)$$

worin $R_1'$ $C_1$-$C_6$-Alkyl bedeutet, umsetzt.

10. Verfahren zur Herstellung von Pyridazinylcarbonsäure-derivaten der Formel I gemäss Anspruch 1, dadurch gekennzeichnet, dass man ein Anilin der Formel II

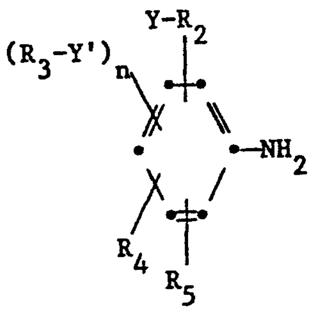

(II)

worin Y, Y', $R_2$, $R_3$, $R_4$, $R_5$ und n die für Formel I angegebenen Bedeutungen haben, in ein entsprechendes Diazoniumsalz der Formel III

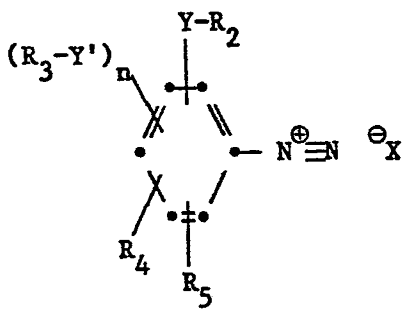

(III)

worin Y, Y', $R_2$, $R_3$, $R_4$, $R_5$ und n die für Formel I angegebenen Bedeutungen haben und X ein geeignetes Anion bedeutet, überführt und dieses Diazoniumsalz mit dem Pyron der Formel IV

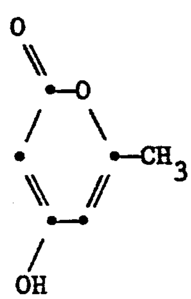

(IV)

oder einem Salz davon umsetzt und das erhaltene Hydrazon der Formel V

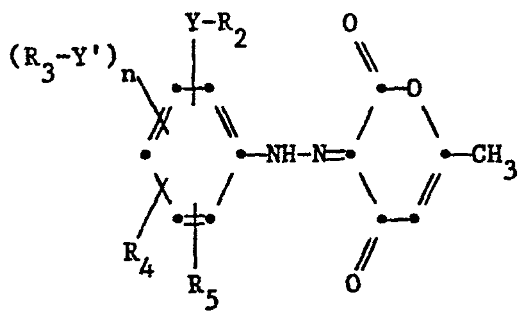 (V)

worin Y, Y', $R_2$, $R_3$, $R_4$, $R_5$ und n die für Formel I angegebenen Bedeutungen haben, durch Zugabe einer Säure, einer Base oder einem Amin, gegebenenfalls unter Umsetzung mit einem Alkohol der Formel VI

$$R_1'-OH \qquad (VI)$$

worin $R_1'$ $C_1$-$C_6$-Alkyl bedeutet, zum Pyridazinylcarbonsäurederivat der Formel I umlagert und gegebenenfalls, wenn die Umlagerung ohne Umsetzung mit einem Alkohol der Formel VI erfolgt und in der Formel I $R_1$ für $C_1$-$C_6$-Alkyl steht, die erhaltene Verbindung in den entsprechenden Alkylester überführt.

11. Gametozides Mittel, welches als Wirkstoff eine gametozid wirksame Menge eines Pyridazinylcarbonsäure-derivats der Formel I gemäss Anspruch 1 enthält.

12. Verfahren zur Regulation von Blütenbildung und -ausgestaltung, dadurch gekennzeichnet, dass man Blütenpflanzen, Teile davon, deren Samen oder deren Standort mit einer Verbindung der Formel I gemäss Anspruch 1 behandelt.

13. Verfahren nach Anspruch 12, dadurch gekennzeichnet, dass man männlich sterile, jedoch fortpflanzungsfähige Pflanzen erzeugt.

14. Verfahren nach Anspruch 13, dadurch gekennzeichnet, dass man Kulturpflanzen behandelt.

15. Verfahren nach Anspruch 14, dadurch gekennzeichnet, dass man die Behandlung an kleinkörnigen Halmfruchtpflanzen, Getreidepflanzen oder Futtergräsern nach dem Auflaufen der Pflanzen, aber vor dem Sichtbarwerden von Aehren und Antheren durchführt.

16. Verfahren nach Anspruch 15, dadurch gekennzeichnet, dass man die Behandlung im 5 1/2-Blattstadium durchführt.

17. Verfahren nach Anspruch 12, dadurch gekennzeichnet, dass man die Applikation an Kulturpflanzen vor Blühbeginn durchführt.

18. Verfahren nach Anspruch 17, dadurch gekennzeichnet, dass man die Behandlung an Sonnenblumen-, Baumwoll-, Kürbis-, Gurken-, Melonen-, Hülsenfrucht- oder Zierpflanzen durchführt.

19. Verfahren zur Gewinnung von Hybrid-Samen, dadurch gekennzeichnet, dass man eine als weibliche Elternpflanze ausgewählte Stammpflanze zu Beginn der Blütenbildung, aber vor Ausbildung der männlichen Blütenteile, durch Applikation einer Verbindung der Formel I gemäss Anspruch 1 männlich sterilisiert und anschliessend mit Pollen einer ausgewählten genetisch ähnlichen Pflanze bestäubt und den reifen Hybrid-Samen erntet.

20. Verfahren nach Anspruch 19, dadurch gekennzeichnet, dass man den Wirkstoff in einer Aufwandmenge von 0,05 bis 12 kg Aktivsubstanz pro Hektar auf die weibliche Elternpflanze oder deren Standort appliziert.

FO 7.5 SES/eg*/jt*

Patentansprüche für den Vertragsstaat AT

1. Gametozides Mittel, welches als Wirkstoff eine gametozid wirksame Menge eines Pyridazinylcarbonsäure-derivats der Formel I

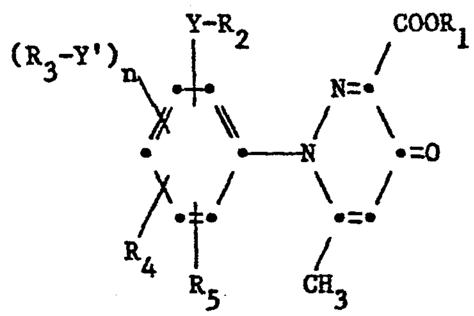

(I)

enthält, worin

$R_1$ Wasserstoff, ein Kation oder $C_1$-$C_6$-Alkyl,
Y und Y' unabhängig voneinander ein Sauerstoff- oder Schwefelatom oder eine Sulfinyl- oder Sulfonylgruppe,
$R_2$ und $R_3$ unabhängig voneinander eine ein- oder mehrfach durch Halogen substituierte $C_1$-$C_{12}$-Alkyl- oder $C_1$-$C_{12}$-Alkenylgruppe,
$R_4$ und $R_5$ unabhängig voneinander Wasserstoff, Halogen, Nitro, Cyan, $C_1$-$C_6$-Alkoxy oder unsubstituiertes oder ein- oder mehrfach durch Halogen substituiertes $C_1$-$C_6$-Alkyl und n 0 oder 1 bedeutet.

2. Gametozides Mittel nach Anspruch 1, welches als Wirkstoff ein Pyridazinylcarbonsäure-derivat der Formel I enthält, worin $R_1$ Wasserstoff, ein Alkalimetall- oder Erdalkalimetallkation, das Ammoniumkation oder $C_1$-$C_4$-Alkyl, Y und Y' unabhängig voneinander ein Sauerstoff- oder Schwefelatom oder eine Sulfonylgruppe, $R_2$ und $R_3$ unabhängig voneinander eine durch mindestens zwei Fluoratome substituierte $C_1$-$C_3$-Alkylgruppe oder eine durch mindestens zwei Chloratome substituierte $C_2$-$C_4$-Alkenylgruppe, $R_4$ Wasserstoff, Halogen, Nitro oder $C_1$-$C_4$-Alkyl, $R_5$ Wasserstoff und n 0 oder 1 bedeuten.

3. Gametozides Mittel nach Anspruch 2, welches als Wirkstoff ein Pyridazinylcarbonsäure-derivat der Formel I enthält, worin $R_1$ Wasserstoff, das Natrium-, Kalium-, Magnesium- oder Ammoniumkation oder $C_1$-$C_4$-Alkyl, Y ein Sauerstoff- oder Schwefelatom oder eine Sulfonylgruppe, $R_2$ Trifluormethyl, Difluormethyl, 1,1,2,2,2-Pentafluoräthyl, 1,1,2,2-Tetrafluoräthyl, 1,2-Dichlorvinyl oder 1,2,2-Trichlorvinyl, Y' ein Sauerstoffatom, $R_3$ Difluormethyl, $R_4$ Wasserstoff, Chlor, Nitro oder Methyl, $R_5$ Wasserstoff und n 0 oder 1 bedeuten.

4. Gametozides Mittel nach Anspruch 3, welches als Wirkstoff ein Pyridazinylcarbonsäure-derivat der Formel I enthält, worin $R_1$ Wasserstoff, das Natriumkation, Methyl oder Aethyl, Y ein Sauerstoff- oder Schwefelatom, $R_2$ Trifluormethyl, Difluormethyl, 1,1,2,2-Tetrafluoräthyl, 1,1,2,2,2-Pentafluoräthyl oder 1,2-Dichlorvinyl, $R_4$ Wasserstoff oder Methyl, $R_5$ Wasserstoff und n 0 bedeuten.

5. Gametozides Mittel nach Anspruch 4, welches als Wirkstoff ein Pyridazinylcarbonsäure-derivat der Formel I enthält, worin $R_1$ Wasserstoff, Methyl oder Aethyl, Y ein Sauerstoff- oder Schwefelatom, $R_2$ Trifluormethyl, Difluormethyl, 1,1,2,2-Tetrafluoräthyl oder 1,1,2,2,2-Pentafluoräthyl, $R_4$ Wasserstoff oder Methyl, $R_5$ Wasserstoff und n 0 bedeuten.

6. Gametozides Mittel nach Anspruch 5, welches als Wirkstoff ein Pyridazinylcarbonsäure-derivat der Formel I enthält, worin $R_1$ für Wasserstoff, Methyl oder Aethyl, Y für ein Sauerstoffatom, $R_2$ für Trifluormethyl oder Difluormethyl, $R_4$ für Wasserstoff oder Methyl, $R_5$ für Wasserstoff und n für 0 steht.

7. Gametozides Mittel nach Anspruch 5, welches als Wirkstoff ein Pyridazinylcarbonsäure-derivat der Formel I enthält, ausgewählt aus der Gruppe

[1-(4-(1,1,2,2-Tetrafluoräthoxy)-phenyl)-1,4-dihydro-4-oxo-6-methyl-pyridazin-3-yl]-carbonsäure,

[1-(3-Methyl-4-(1,1,2,2-tetrafluoräthoxy)-phenyl)-1,4-dihydro-4-oxo-6-methyl-pyridazin-3-yl]-carbonsäure,
[1-(4-Trifluormethylthio)-phenyl)-1,4-dihydro-4-oxo-6-methyl-pyridazin-3-yl]-carbonsäure,
[1-(4-Difluormethylthio)-phenyl)-1,4-dihydro-4-oxo-6-methyl-pyridazin-3-yl]-carbonsäure,
[1-(4-(1,1,2,2,2-Pentafluoräthoxy)-phenyl)-1,4-dihydro-4-oxo-6-methyl-pyridazin-3-yl]-carbonsäure,
[1-(4-(Trifluormethoxy)-phenyl)-1,4-dihydro-4-oxo-6-methyl-pyridazin-3-yl]-carbonsäure,
[1-(4-(Difluormethoxy)-phenyl)-1,4-dihydro-4-oxo-6-methyl-pyridazin-3-yl]-carbonsäure,
[1-(2-Methyl-4-(difluormethoxy)-phenyl)-1,4-dihydro-4-oxo-6-methyl-pyridazin-3-yl]-carbonsäure,
[1-(4-Trifluormethoxy)-phenyl)-1,4-dihydro-4-oxo-6-methyl-pyridazin-3-yl]-carbonsäure-methylester und
[1-(4-(Trifluormethoxy)-phenyl)-1,4-dihydro-4-oxo-6-methyl-pyridazin-3-yl]-carbonsäure-äthylester.

8. Verfahren zur Herstellung von Pyridazinylcarbonsäure-derivaten der Formel I gemäss Anspruch 1, dadurch gekennzeichnet, dass man ein Hydrazon der Formel V

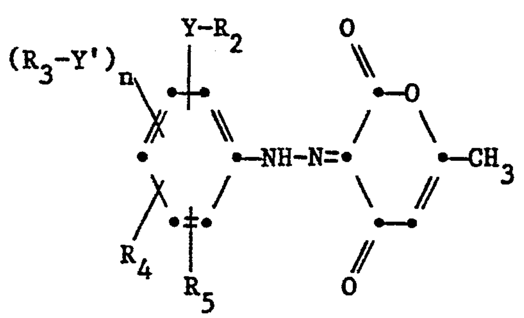

(V)

worin Y, Y', $R_2$, $R_3$, $R_4$, $R_5$ und n die für Formel I angegebenen Bedeutungen haben, durch Zugabe einer Säure, einer Base oder einem Amin zum Pyridazinylcarbonsäure-derivat der Formel I umlagert und gegebenenfalls, wenn $R_1$ in der Formel I für $C_1$-$C_6$-Alkyl steht, die erhaltene Verbindung in den entsprechenden Alkylester überführt.

9. Verfahren nach Anspruch 8, dadurch gekennzeichnet, dass man ein Hydrazon der Formel V mit einem Alkohol der Formel VI

$$R_1' - OH \qquad (VI)$$

worin $R_1'$ $C_1$-$C_6$-Alkyl bedeutet, umsetzt.

10. Verfahren zur Herstellung von Pyridazinylcarbonsäure-derivaten der Formel I gemäss Anspruch 1, dadurch gekennzeichnet, dass man ein Anilin der Formel II

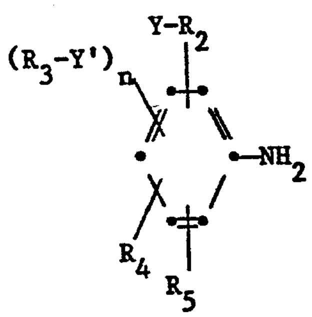

(II)

worin Y, Y', $R_2$, $R_3$, $R_4$, $R_5$ und n die für Formel I angegebenen Bedeutungen haben, in ein entsprechendes Diazoniumsalz der Formel III

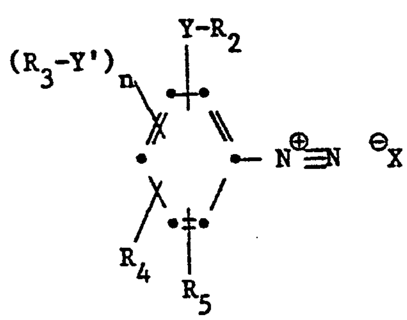

(III)

worin Y, Y', $R_2$, $R_3$, $R_4$, $R_5$ und n die für Formel I angegebenen Bedeutungen haben und X ein geeignetes Anion bedeutet, überführt und dieses Diazoniumsalz mit dem Pyron der Formel IV

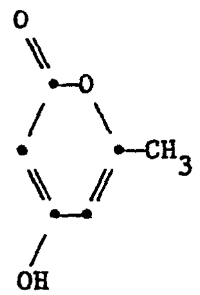

(IV)

oder einem Salz davon umsetzt und das erhaltene Hydrazon der Formel V

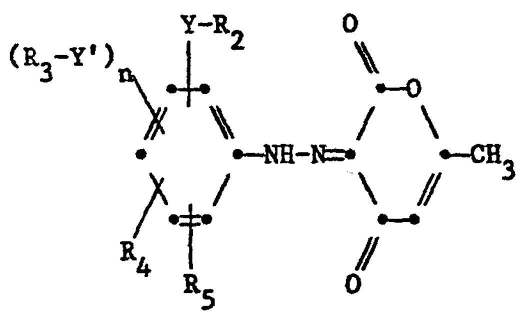

(V)

worin Y, Y', $R_2$, $R_3$, $R_4$, $R_5$ und n die für Formel I angegebenen Bedeutungen haben, durch Zugabe einer Säure, einer Base oder einem Amin, gegebenenfalls unter Umsetzung mit einem Alkohol der Formel VI

$$R_1'-OH \qquad (VI)$$

worin $R_1'$ $C_1$-$C_6$-Alkyl bedeutet, zum Pyridazinylcarbonsäurederivat der Formel I umlagert und gegebenenfalls, wenn die Umlagerung ohne Umsetzung mit einem Alkohol der Formel VI erfolgt und in der Formel I $R_1$ für $C_1$-$C_6$-Alkyl steht, die erhaltene Verbindung in den entsprechenden Alkylester überführt.

11. Verfahren zur Regulation von Blütenbildung und -ausgestaltung, dadurch gekennzeichnet, dass man Blütenpflanzen, Teile davon, deren Samen oder deren Standort mit einer Verbindung der Formel I gemäss Anspruch 1 behandelt.

12. Verfahren nach Anspruch 11, dadurch gekennzeichnet, dass man männlich sterile, jedoch fortpflanzungsfähige Pflanzen erzeugt.

13. Verfahren nach Anspruch 12, dadurch gekennzeichnet, dass man Kulturpflanzen behandelt.

14. Verfahren nach Anspruch 13, dadurch gekennzeichnet, dass man die Behandlung an kleinkörnigen Halmfruchtpflanzen, Getreidepflanzen oder Futtergräsern nach dem Auflaufen der Pflanzen, aber vor dem Sichtbarwerden von Aehren und Antheren durchführt.

15. Verfahren nach Anspruch 14, dadurch gekennzeichnet, dass man die Behandlung im 5 1/2-Blattstadium durchführt.

16. Verfahren nach Anspruch 11, dadurch gekennzeichnet, dass man die Applikation an Kulturpflanzen vor Blühbeginn durchführt.

17. Verfahren nach Anspruch 16, dadurch gekennzeichnet, dass man die Behandlung an Sonnenblumen-, Baumwoll-, Kürbis-, Gurken-, Melonen-, Hülsenfrucht- oder Zierpflanzen durchführt.

18. Verfahren zur Gewinnung von Hybrid-Samen, dadurch gekennzeichnet, dass man eine als weibliche Elternpflanze ausgewählte Stammpflanze zu Beginn der Blütenbildung, aber vor Ausbildung der männlichen Blütenteile, durch Applikation einer Verbindung der Formel I gemäss Anspruch 1 männlich sterilisiert und anschliessend mit Pollen einer ausgewählten genetisch ähnlichen Pflanze bestäubt und den reifen Hybrid-Samen erntet.

19. Verfahren nach Anspruch 18, dadurch gekennzeichnet, dass man den Wirkstoff in einer Aufwandmenge von 0,05 bis 12 kg Aktivsubstanz pro Hektar auf die weibliche Elternpflanze oder deren Standort appliziert.

FO 7.5 SES/eg*/jt*